# FASCICULE DE BREVET EUROPEEN

(11) **EP 1 898 908 B1**
(45) Date de publication et mention de la délivrance du brevet: **30.03.2011**
(21) Numéro de dépôt: 06778713.5
(22) Date de dépôt: 29.06.2006
(51) Int. Cl.: A61K 31/425, C07D 277/50, A61P 25/28

(54) **NOUVEAUX COMPOSES CHIMIQUES ET LEURS UTILISATIONS COMME MÉDICAMENT**
NEUE CHEMISCHE VERBINDUNGEN UND IHRE VERWENDUNGEN ALS MEDIKAMENT
NOVEL CHEMICAL COMPOUNDS AND THE USES THEREOF AS A MEDICAMENT

(30) Priorité: 01.07.2005 FR 0507023
(43) Date de publication de la demande: 19.03.2008
(73) Titulaire: Trophos, 13288 Marseille Cedex 9 (FR)
(72) Inventeur: CHAIMBAULT, Corinne, F-13008 Marseille (FR); DROUOT, Cyrille, F-13009 Marseille (FR); JAMOT, Laure, F-75013 Paris (FR); PRUSS, Rebecca, F-13260 Cassis (FR); SIMON, Céline, F-67400 Illkirch Graffenstaden (FR)
(74) Mandataire: Galup, Cédric Olivier Nicolas
(86) Numéro de dépôt international: PCT/FR2006/001521
(87) Numéro de publication internationale: WO 2007/003767

(56) Documents cités:
- WO-A-03/006426
- WO-A-2004/033439
- WO-A-2004/063196
- DATABASE CA [Online] CHEMICAL ABSTRACTS SERVICE, COLUMBUS, OHIO, US; AMAL, HAYRIYE ET AL: "Synthesis of some antipyrylthiazolylhydrazones. III" XP002374078 extrait de STN Database accession no. 1982:472283 & JOURNAL OF FACULTY OF PHARMACY OF ISTANBUL UNIVERSITY , 17, 91-102 CODEN: IEFMA9; ISSN: 0367-7524, 1981,
- DATABASE CA [Online] CHEMICAL ABSTRACTS SERVICE, COLUMBUS, OHIO, US; ATES, OZNUR: "Antipyrine 4-methylthiazolonylhydrazones. III" XP002374081 extrait de STN Database accession no. 1974:425599 & JOURNAL OF FACULTY OF PHARMACY OF ISTANBUL UNIVERSITY , 9(1), 43-50 CODEN: IEFMA9; ISSN: 0367-7524, 1973,
- DATABASE CA [Online] CHEMICAL ABSTRACTS SERVICE, COLUMBUS, OHIO, US; GURSOY, AYSEL: "Coumarinylthiazolylhydrazone derivatives. I" XP002374082 extrait de STN Database accession no. 1973:442397 & JOURNAL OF FACULTY OF PHARMACY OF ISTANBUL UNIVERSITY , 8(2), 75-88 CODEN: IEFMA9; ISSN: 0367-7524, 1972,
- DATABASE CA [Online] CHEMICAL ABSTRACTS SERVICE, COLUMBUS, OHIO, US; ERGENC, NEDIME ET AL: "Reactions of some thiosemicarbazones with chloromethyl antipyrinyl ketone. II" XP002374083 extrait de STN Database accession no. 1971:420284 & JOURNAL OF FACULTY OF PHARMACY OF ISTANBUL UNIVERSITY , 6(2), 80-5 CODEN: IEFMA9; ISSN: 0367-7524, 1970,
- DATABASE CA [Online] CHEMICAL ABSTRACTS SERVICE, COLUMBUS, OHIO, US; MEDNE, A. ET AL: "Synthesis and transformations of furan derivatives. V. Synthesis of 4-methyl-2-thiazolylhydrazones of aldehydes and ketones of the furan series" XP002374085 extrait de STN Database accession no. 1966:4133 & KHIMIYA GETEROTSIKLICHESKIKH SOEDINENII , (4), 629-31 CODEN: KGSSAQ; ISSN: 0132-6244, 1965,

## Description

La présente invention se place dans le domaine de la pharmacie, particulièrement dans le domaine du traitement des maladies neurodégénératives. Très particulièrement elle concerne une famille de composés chimiques pour lesquels une activité neuroprotectrice a pu être démontrée.

Certains membres de ladite famille étant des composés nouveaux jamais décrits, l'invention concerne ces produits nouveaux, leur procédé de synthèse ainsi que certains intermédiaires de synthèse eux-mêmes nouveaux.

La présente invention concerne également des compositions comprenant les composés de la famille, l'utilisation desdits composés à titre de médicaments particulièrement dans la préparation d'un médicament destiné au traitement des maladies neurodégénératives.

Les processus neurodégénératifs sont caractérisés par le dysfonctionnement et la mort des neurones entraînant la perte des fonctions neurologiques médiées par le cerveau, la moëlle épinière (système nerveux central, SNC) et le système nerveux périphérique (SNP). Ils peuvent résulter, entre autres, de traumatisme, d'exposition à des toxines ou de situations pathologiques regroupées sous le terme de maladies ou affections neurodégénératives.

Sans prétendre être exhaustif, on peut citer parmi les pathologies les plus importantes qui sont caractérisées par un processus dégénératif :
- les maladies chroniques neurodégénératives, héréditaires ou sporadiques, notamment la maladie d'Alzheimer (AD), la maladie de Huntington (HD), la maladie de Parkinson (PD), la sclérose latérale amyotrophique (ALS), les amyotrophies spinales (SMA), la maladie de Creutzfeldt-Jakob, la sclérose en plaque (MS), l'adrénoleucodystrophie, l'épilepsie, les démences, la schizophrénie, les syndromes neurologiques associés au syndrome d'immuno-déficience acquis (SIDA) ;
- les lésions neuronales liées au vieillissement ;
- les neuropathies périphériques héréditaires ou lésionnelles, comme les maladies de Fabry, de Charcot-Marie-Tooth, de Krabbe, les leucodystrophies, les neuropathies diabétiques et celles induites par les traitements anti-cancéreux ;
- les traumatismes du cerveau, des nerfs périphériques ou de la moëlle épinière ;
- les ischémies du cerveau ou de la moëlle épinière suite à un accident cérébro-vasculaire, ou induites par un manque d'irrigation sanguine ;
- les dégénérescences héréditaires, lésionnelles ou liées au vieillissement des neurones sensoriels de la vision, comme les dégénérescences maculaires, les rétinites pigmentaires, ou les dégénérescences du nerf optique induites par les glaucomes ;
- les dégénérescences héréditaires, traumatiques ou liées au vieillissement des neurones sensoriels de l'ouïe entraînant une diminution ou une perte de l'audition.

Un grand nombre de maladies neurodégénératives sont caractérisées par l'apparition de mutations dans certaines protéines ayant pour conséquence une altération de la conformation desdites protéines. Ces mutations sont souvent responsables d'un gain ou d'une perte d'une ou plusieurs fonctions liées à l'expression de ces protéines mutées.

L'altération structurelle des protéines mutées entraîne la formation d'agrégats insolubles, ubiquitinylés, dans les cellules affectées comme cela a été décrit pour la maladie de Parkinson (PD), pour les démences à corps de Lewy *(Dementia with Lewy bodies),* pour la maladie de Huntington (HD), pour des ataxies spino-cérébelleuses (SCA, *spino-cerebral atrophy,),* pour la maladie de Kennedy ou atrophie musculaire spino-bulbaire *(spino-bulbar muscular atrophy,* SBMA), pour la sclérose latérale amyotrophique (ALS), ou en dehors de ces cellules comme cela a été décrit pour la maladie d'Alzheimer (AD).

Cette description est plus particulièrement correcte pour les maladies neurodégénératives causées par l'expansion de codons CAG (codant la glutamine) dans la partie codante ou régulatrice d'un gène. Par ailleurs dans le texte, ces maladies neurodégénératives pourront être désignées sous le nom de "dégénérescences neuronales à polyglutamines". En effet de telles mutations induisent des modifications des interactions protéiques normalement mises en oeuvre par la protéine non mutée ; interactions qui régulent l'activité neuronale dans son ensemble. De telles maladies comprennent mais ne sont pas restreintes à :
- la maladie d'Huntington, où de multiples répétitions de codons "glutamine" (plus de 36 répétitions) dans le gène IT-15 codant la protéine huntingtine, cause la perte de neurones du striatum et du cortex,
- la maladie de Kennedy (ou atrophie musculaire spino-bulbaire, SBMA) où de multiples répétitions de codons "glutamine" (plus de 40 codons) dans le gène du récepteur aux androgènes, cause la perte de moto-neurones lombaires,
- l'ataxie spino-cérébelleuse (SCA, *spino-cerebral atrophy)* de type 1 (ou SCA-1) où de multiples répétitions de l'acide aminé glutamine (plus de 41 acides aminés) dans l'ataxine-1, cause la perte de neurones du cervelet et du tronc cérébral,
- la maladie de Macchado-Joseph (ou SCA-3) où de multiples répétitions de l'acide aminé glutamine (plus de 68 acides aminés) dans la protéine MJD-1, cause la perte de neurones de la moëlle épinière et du cervelet,
- la SCA-6 où les répétitions polymorphes de CAG s'expriment dans le canal calcium voltage-dépendant alpha 1A,
- la SCA-7 dans laquelle la mutation portée sur l'ataxine-7 est associée, outre à la dégénérescence cérébro-spinale, à une dégénérescence rétinienne,
- l'atrophie dentato-rubro-pallido-luysienne (DRPLA) où de multiples répétitions de codons "glutamine" (plus de 49 codons) dans la partie codante de l'atrophine est la cause de la perte de neurones du globus pallidus et des noyaux dentato-rubral et subthalamiques.

On peut encore citer d'autres atrophies spinocérébelleuses, caractérisées par une dégénérescence de la moëlle épinière et d'autres régions cérébrales, comme par exemple
o la SCA-2 dont la mutation est portée sur le chromosome 12,
o l'ataxie de Friedreich portée sur le chromosome 9 avec de multiples répétitions de codons GAA au sein d'un intron, perturbant l'expression de la frataxine (protéine mitochondriale)
o l'ataxie avec déficience sélective en vitamine E portée sur le chromosome 8, s'exprimant par une carence en alpha-TTP *(Tocopherol Transfer Protein)* (déficience en vitamine E),
o l'épilepsie myoclonique à fibres disparates qui s'exprime par une mutation de l'ADN mitochondrial,
o le syndrome d'encéphalopathie mitochondriale,
o l'ataxie télangiectasie portée sur le chromosome 11 dans le gène ATM et
o l'ataxie cérébelleuse juvénile de Nikali, portée sur le chromosome 10.

Une approche thérapeutique de ces pathologies passe par la neuroprotection, c'est-à-dire le maintien des cellules nerveuses dans leur état physiologique naturel, voire le rétablissement d'un état physiologique normal chez des cellules nerveuses pathologiques.

Une des approches thérapeutiques pour protéger tes neurones de la mort, décrites dans l'art antérieur, est l'apport de protéines neurotrophiques. Ces protéines, telles que le facteur neurotrophique dérivé du cerveau (brain-derived neurotrophic factor, BDNF), le facteur neurotrophique ciliaire (ciliary neurotrophic factor CNTF), le facteur de croissance des nerfs (nerve growth factor NGF), le facteur neurotrophique dérivé de la glie (glia-derived neurotrophic factor GDNF) sont synthétisées au cours du développement embryonnaire ou après lésion chez l'adulte. Ces facteurs de croissance favorisent la survie, la maturation et la différentiation des cellules neuronales. De plus, ils inhibent les mécanismes apoptotiques, activent de multiples voies de survie et protègent un grand nombre de populations neuronales.

L'art antérieur propose donc l'utilisation de ces facteurs de croissance dans le traitement de la plupart des dégénérescences neuronales. Mais d'autres voies sont également décrites dans l'art antérieur, comme par exemple la correction des dysfonctionnements neuronaux précoces par une action qui n'implique pas de mimétisme trophique.

Cependant, et sans dénigrer les efforts de la science afin de faire progresser les traitements des maladies neurodégénératives, il n'existe pas actuellement de traitement pleinement efficace pour enrayer les dégénérescences neuronales, particulièrement les dégénérescences neuronales à polyglutamine.

Toutes les pathologies neurodégénératives, particulièrement celles décrites précédemment, devraient répondre à un traitement neuroprotecteur administré à un stade précoce de la maladie par une amélioration durable des fonctions neuronales préservées et une réduction de la progression de la pathologie.

La demanderesse, après de longues recherches, a mis en évidence les propriétés thérapeutiques notamment dans le traitement ou la prévention des affections neurodégénératives, de composés chimiques répondant à la formule I, dont certains sont des composés nouveaux.

Ainsi l'invention a pour objet premier des composés chimiques nouveaux répondant à la formule I 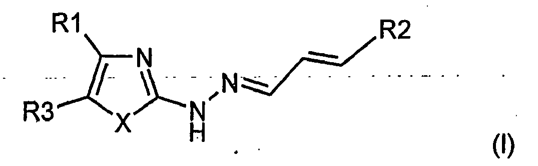 dans laquelle
X représente un hétéroatome choisi parmi l'oxygène, le soufre ou l'azote, l'atome d'azote étant éventuellement substitué par un groupement alkyle ;
R1 et R2 représentent un groupement alkyle, cycloalkyle, aryle ou hétéroaryle ;
R3 représente un atome d'hydrogène ou un groupement alkyle ou aryle,
à l'exception des composés
- 3-phényl-2-propénal-[4-(4-nitrophényl)-2-thiazolyl] hydrazone ;
- 3-phényl-2-propénal-[4-(4-méthoxyphényl)-2-thiazolyl] hydrazone ;
- 3-phényl-2-propénal-[4-(4-fluorophényl)-2-thiazolyl] hydrazone ;
- 3-phényl-2-propénal-[4-(4-bromophényl)-2-thiazolyl] hydrazone ;
- 3-phényl-2-propénal-[4-(4-chlorophényl)-2-thiazolyl] hydrazone ;
- 3-phényl-2-propénal-[4-(4-méthylphényl)-2-thiazolyl] hydrazone ;
- 3-phényl-2-propénal- [4-(1-naphthalènyl)-2-thiazolyl] hydrazone ;
- 3-phényl-2-propénal-(5-méthyl-4-phényl-2-thiazolyl) hydrazone ;
- 3-phényl-2-propénal-[4-(2,4-dichlorophényl)-2-thiazolyl] hydrazone ;
- 3-phényl-2-propénal-[4-(3-nitrophényl)-2-thiazolyl] hydrazone ;
- 3-phényl-2-propénal-[4-(1,1-diméthyléthyl)-2-thiazolyl] hydrazone ;
- 3-phényl-2-propénal-[4-(4-chlorophényl)-5-phényl-2-thiazolyl] hydrazone ;
- 2-buténal-[4-(4-nitrophényl)-2-thiazolyl] hydrazone ;
- 3-phényl-2-propénal-[5-(4-chlorophényl)-4-phényl-2-thiazolyl] hydrazone ;
- 3-(4-nitrophényl)-2-propénal-(4,5-diphényl-2-thiazolyl) hydrazone ;
- 3-phényl-2-propénal-(4,5-diphényl-2-thiazolyl) hydrazone ;
- acide 2-[[3-(3-nitrophényl)-2-propénylidène]hydrazino]-4-thiazole carboxylique
- acide 2-[[3-(2-nitrophényl)-2-propénylidène]hydrazino]-4-thiazole carboxylique
- 3-phényl-2-propénal-[4-chlorométhyl)-2-thiazolyl] hydrazone ;
- cinnamaldéhyde-(4-phényl-2-thiazolyl) hydrazone ;
- crotonaldéhyde-(4-phényl-2-thiazolyl) hydrazone ;
- 2-furanacroléine-(4-méthyl-2-thiazolyl) hydrazone ;
- cinnamaldéhyde-(4-méthyl-2-thiazolyl) hydrazone ;
ainsi que leurs sels d'addition avec les acides pharmaceutiquement acceptables.

Selon l'invention, les sels d'addition avec les acides pharmaceutiquement acceptantes peuvent être par exemple des sels formés avec les acides chlorhydrique, bromhydrique, nitrique, sulfurique, phosphorique, ascétique, formique, propionique, benzoïque, maléique, fumarique, succinique, tartrique, citrique, oxalique, glyoxylique, aspartique, alcanes sulfoniques tels que les acides méthane ou éthane sulfoniques, arylsulfoniques, tels que les acides benzène ou paratoluène sulfoniques, ou carboxyliques. Préférentiellement selon l'invention, les sels d'addition sont le chlorhydrate, le tartrate, le méthane sulfonate.

Selon l'invention on entend par
- Groupement alkyle, un radical carboné ayant de 1 à 6 atomes de carbone, linéaire ou ramifié, éventuellement substitué par un atome d'halogène, un radical hydroxyle, un groupement amino, un groupement acide carboxylique. Par radical carboné ayant de 1 à 6 atomes de carbone, linéaire ou ramifié, on entend un radical choisi parmi les radicaux méthyle, éthyle, propyle, isopropyle, butyle, isobutyle, secbutyle, tertiobutyle, pentyle, hexyle. Selon l'invention un groupement alkyle préféré est un radical méthyle.
- Groupement cycloalkyle, un radical dérivé d'un noyau carboné saturé cyclique ayant de 3 à 6 atomes de carbone, éventuellement substitué par un ou plusieurs atomes d'halogène, par un ou plusieurs radicaux alkyles ayant de 1 à 4 atomes de carbone, linéaire ou ramifié.

Par radical dérivé d'un noyau carboné saturé cyclique ayant de 3 à 6 atomes de carbone, on entend un radical choisi parmi les radicaux cyclopropyle, cyclobutyle, cyclopentyle et cyclohexyle. Selon l'invention un groupement cycloalkyle préféré est un radical cyclohexyle.
- Groupement aryle, un radical dérivé d'un noyau carboné aromatique cyclique ou polycyclique ayant de 6 à 10 atomes de carbone, éventuellement substitué une ou plusieurs fois. Les substituants peuvent être choisis parmi un atome d'halogène, un radical alkyle ayant de 1 à 4 atomes de carbone, linéaire ou ramifié, éventuellement substitué par un atome ou plusieurs atomes d'halogène, par un radical hydroxyle, par un groupement amino, ou par un radical alkoxy ayant de 1 à 3 atomes de carbone, un radical hydroxyle, un groupement nitro, un groupement cyano, un groupement 1,3-dioxolyle, un groupement carbonyle, un groupement méthylsulfone ou un groupement amino éventuellement mono ou disubstitué par une chaîne alkyle de 1 à 3 atomes de carbone ; préférentiellement selon l'invention le groupement aryle est un phényle éventuellement substitué comme indiqué précédemment ;
- Groupement hétéroaryle, un radical mono cyclique aromatique dont le cycle présente 5 ou 6 atomes et comportant soit 1 ou 2 atomes d'azote soit 1 atome d'oxygène. Préférentiellement selon l'invention, le groupement hétéroaryle préféré est choisi parmi les groupements pyrimidine et pyridine.
- Groupement carbonyle, un groupement aldéhyde, alkylcétone, amide éventuellement substitué par une chaîne alkyle, acide carboxylique ou ester d'alkyle.

Selon une forme préférentielle de l'invention, les composés de formule I sont ceux dans lesquels X représente un atome de soufre.

Selon une autre forme préférentielle de l'invention, les composés de formule I sont ceux pour lesquels, toute chose étant identique par ailleurs à ce qui est précédemment décrit, R1 est un radical phényle substitué en position para par un atome de chlore, par un groupement méthyle ou trifluorométhyle, ou en position méta par un groupement hydroxyle, ou un radical phényle disubstitué en ortho par un groupement méthoxy et en para par un atome de chlore, ou en ortho par un groupement méthyle et en para par un groupement hydroxyle, ou en méta par un atome de fluor et en para par un atome de chlore.

Selon encore une autre forme préférentielle de l'invention, les composés de formule I sont ceux pour lesquels, toute chose étant identique par ailleurs à ce qui est précédemment décrit, R2 est un radical pyridin-4-yle, pyridin-2-yle ou pyridin-3-yle, ou un radical benzo[1,3]dioxol-5-yle, ou un radical phényle, ou phényle substitué par un groupement nitro, cyano, méthoxy, amino, méthylsulfone, amide, méthylcétone, hydroxyméthyle ou hydroxyle en position méta, ou phényle substitué en position ortho par un groupement méthoxy, méthyle, un atome de fluor ou de chlore, ou phényle substitué en position para par un atome de fluor.

Selon encore une autre forme préférentielle de l'invention, les composés de formule I sont ceux dans lesquels, toute chose étant identique par ailleurs à ce qui est précédemment décrit, R3 est un atome d'hydrogène ou un groupement méthyle.

Selon une forme encore plus préférentielle de l'invention, les composés de formule I sont ceux dans lesquels
X représente un atome de soufre,
R3 est un atome d'hydrogène,
R1 est un radical phényle substitué en position para par un atome de chlore, par un groupement trifluorométhyle, ou en position méta par un groupement hydroxyle, ou un radical phényle disubstitué en ortho par un groupement méthoxy et en para par un atome de chlore, ou en ortho par un groupement méthyle et en para par un groupement hydroxyle, ou en méta par un atome de fluor et en para par un atome de chlore,
R2 est un radical pyridin-4-yle, pyridin-2-yle ou pyridin-3-yle, ou un radical benzo[1,3]dioxol-5-yle, ou un radical phényle, ou phényle substitué par un groupement nitro, cyano, amino, méthylsulfone, amide, méthylcétone, hydroxyméthyle, hydroxyle ou méthoxy en position méta, ou phényle substitué en position ortho par un groupement méthoxy, méthyle, un atome de fluor ou de chlore, ou phényle substitué en position para par un atome de fluor.

Ainsi les composés nouveaux préférés selon l'invention sont .
- la 3-(2-fluorophényl)-2-propénal-[4-(4-chlorophényl-2-thiazolyl) hydrazone ;
- la 3-(3-nitrophényl)-2-propénal-[4-(4-chlorophényl-2-thiazolyl) hydrazone ;
- la 3-(2-méthoxyphényl)-2-propénal-[4-(4-chlorophényl-2-thiazolyl) hydrazone ;
- la 3-(3-méthoxyphényl)-2-propénal-[4-(4-chlorophényl-2-thiazolyl) hydrazone ;
- la 3-phényl-2-propénal-[4-(3-hydroxyphényl-2-thiazolyl) hydrazone ;
- la 3-pyridin-4-yle-2-propénal-[4-(4-chlorophényl-2-thiazolyl) hydrazone;
- la 3-(2-méthylphényl)-2-propénal-[4-(4-chlorophényl-2-thiazolyl) hydrazone ;
- la 3-(3-cyanophény)-2-propénal-[4-(4-ch)orophényl-2-thiazolyl) hydrazone ;
- la 3-(2-chlorophényl)-2-propénal-[4-(4-chlorophényl-2-thiazolyl) hydrazone ;
- la 3-(3-aminophényl)-2-propénal-[4-(4-chlorophényl-2-thiazolyl) hydrazone ;
- la 3-(3-nitrophényl)-2-propénal-[4-(4-trifluorométhylphényl-2-thiazolyl) hydrazone ;
- le 3-((E)-3-{[4-(4-chlorophényl)-thiazol-2-yl]-hydrazono}-propényl)-phénol ;
- la 3-pyridin-4-yl-2-propénal-[4-(4-trifluorométhylphényl-2-thiazolyl) hydrazone ;
- la 3-(3-aminophényl)-2-propénal-[4-(4-trifluorométhylphényl-2-thiazolyl) hydrazone ;
- la 3-(3-méthanesulfonylphényl)-2-propénal-[4-(4-chlorophényl-2-thiazolyl) hydrazone ;
- la 3-(3-cyanophényl)-2-propénal-[4-(4-hydroxyphényl-2-thiazolyl) hydrazone ;
- la 3-(3-benzo[1,3]dioxol-5-yl)-2-propénal-[4-(4-trifluorométhylphényl-2-thiazolyl) hydrazone ;
- la 3-phényl-2-propénal-[4-(4-chloro-2-méthoxyphényl-2-thiazolyl) hydrazone ;
- la 1-[3-((E)-3-{[4-(4-trifluorométhylphényl)-thiazol-2-yl]-hydrazono}-propényl)-phényl]-éthanone ;
- la 3-pyridin-2-yl-2-propénal-[4-(4-chlorophényl-2-thiazolyl) hydrazone ;
- la 3-pyridin-2-yl-2-propénal-[4-(4-trifluorométhylphényl-2-thiazolyl) hydrazone ;
- le 3-méthyl-4-(2-{N'-[(E)-3-phénylprop-2-èn-(E)-ylidène]-hydrazino}-thiazol-4-yl)-phénol ;
- le [3-((E)-3-([4-(4-trifluorométhylphényl)-thiazol-2-yl]-hydrazono}-propènyl)-phényl]-méthanol ;
- la 3-(4-flurophényl)-2-propénal-[4-(4-trifluorométhylphényl-2-thiazolyl) hydrazone ;
- la 3-((E)-3-{[4-(4-trifluorométhylphényl)-thiazol-2-yl]-hydrazono}-propènyl)-benzamide ;
et tout particulièrement la 3-phényl-2-propénal-[4-(4-trifluorométhylphényl)-2-thiazolyl]hydrazone ; le 3-((E)-3-{[4-(4-trifluorométhylphényl)-thiazol-2-yl]-hydrazono}-propényl)-phénol ; la 3-(3-méthanesulfonylphényl)-2-propénal-[4-(4-trifluorométhylphényl-2-thiazolyl)hydrazone ; et la 3-(3-cyanophényl)-2-propénal-[4-(4-trifluorométhylphényl-2-thiazolyl)hydrazone.

La présente invention a également pour objet des procédés de préparation des nouveaux composés de formule I.

Selon un aspect particulier de l'invention, lorsque le composé de formule I souhaité est un composé dans lequel X représente un atome de soufre, R1, R2 et R3 ayant les significations précédemment décrites, l'invention a pour objet un procédé dans lequel on fait réagir, une α-bromo-cétone de formule II 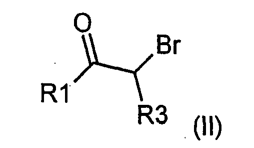 dans laquelle R1 et R3 peuvent avoir les significations précédemment indiquées
avec un composé de formule III, 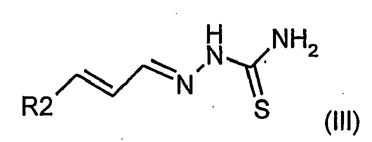 dans laquelle R2 peut avoir la signification précédemment indiquée, pour obtenir le composé de formule I attendu.

Dans les conditions préférentielles de mise en oeuvre du procédé ci-dessus décrit, la réaction du composé de formule II avec le composé de formule III est réalisée en quantité équimolaire
- dans un minimum de solvant adapté, comme par exemple l'éthanol absolu,
- à une température comprise entre 40 et 90°C, préférentiellement entre 50 et 80°C, et pendant un temps compris entre 10 et 30 heures, préférentiellement entre 15 et 20 heures.

Les composés de formule II sont des dérivés connues, décrits (Tetrahedron 2003, 59(8), 1317-1325 ; JOC, 2003, 68(4), 1594-1596) et /ou commerciaux.

Certains composés de formule III, qui servent d'intermédiaires de synthèse, sont nouveaux.

Ledit procédé de synthèse des composés de formule III, est caractérisé en ce que l'on fait réagir un composé de formule IV, 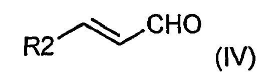 dans laquelle R2 peuvent avoir les significations précédemment indiquées, avec de la thiosemicarbazide pour obtenir le composé nouveau de formule III attendu.

Dans les conditions préférentielles de mise en oeuvre du procédé ci-dessus décrit, la réaction du composé de formule IV est réalisée en quantité équimolaire de thiosemicarbazide dans un minimum de solvant adapté tel que le méthanol anhydre au reflux pendant un temps compris entre 1 heure et 3 heures.

Les composés de formule IV sont des dérivés connus, décrits (Org. Lett. 2003, 5(5) 777-780) et /ou commerciaux.

Selon encore un autre aspect, l'invention a pour objet des compositions, notamment des compositions pharmaceutiques ou médicaments, comprenant au moins un composé de formule I, à l'exception des composés
- 3-phényl-2-propénal-[4-(4-nitrophényl)-2-thiazolyl] hydrazone ;
- 3-phényl-2-propénal-[4-(4-méthoxyphényl)-2-thiazolyl] hydrazone ;
- 3-phényl-2-propénal-[4-(4-fluorophényl)-2-thiazolyl] hydrazone ;
- 3-phényl-2-propénal-[4-(4-bromophényl)-2-thiazolyl] hydrazone ;
- 3-phényl-2-propénal-[4-(4-chlorophényl)-2-thiazolyl] hydrazone ;
- 3-phényl-2-propénal-[4-(4-méthylphényl)-2-thiazolyl] hydrazone ;
- 3-phényl-2-propénal- [4-(1-naphthalènyl)-2-thiazolyl] hydrazone ;
- 3-phényl-2-propénal-(5-méthyl-4-phényl-2-thiazolyl) hydrazone ;
- 3-phényl-2-propénal-[4-(2,4-dichlorophényl)-2-thiazolyl] hydrazone ;
- 3-phényl-2-propénal-[4-(3-nitrophényl)-2-thiazolyl] hydrazone ;
- 3-phényl-2-propénal-[4-(1,1-diméthyléthyl)-2-thiazolyl] hydrazone ;
- 3-phényl-2-propénal-[4-(4-chlorophényl)-5-phényl-2-thiazolyl] hydrazone ;
- 2-buténal-[4-(4-nitrophényl)-2-thiazolyl] hydrazone ;
- 3-phényl-2-propénal-[5-(4-chlorophényl)-4-phényl-2-thiazolyl] hydrazone ;
- 3-(4-nitrophényl)-2-propénal-(4,5-diphényl-2-thiazolyl) hydrazone ;
- 3-phényl-2-propénal-(4,5-diphényl-2-thiazolyl) hydrazone ;
- acide 2-[[3-(3-nitrophényl)-2-propénylidène]hydrazino]-4-thiazole carboxylique
- acide 2-[[3-(2-nitrophényl)-2-propénylidène]hydrazino]-4-thiazole carboxylique
- 3-phényl-2-propénal-[4-chlorométhyl)-2-thiazolyl] hydrazone ;
- cinnamaldéhyde-(4-phényl-2-thiazolyl) hydrazone ;
- crotonaldéhyde-(4-phényl-2-thiazolyl) hydrazone ;
- 2-furanacroléine-(4-méthyl-2-thiazolyl) hydrazone ;
- cinnamaldéhyde-(4-méthyl-2-thiazolyl) hydrazone ;
ainsi que leurs sels d'addition avec les acides pharmaceutiquement acceptables.

Les compositions pharmaceutiques selon l'invention peuvent comprendre en outre au moins un autre ingrédient thérapeutiquement actif, pour une utilisation simultanée, séparée ou étalée dans le temps, notamment lors d'un traitement chez un sujet atteint d'une pathologie neurodégénérative.

Les compositions pharmaceutiques selon l'invention peuvent avantageusement comprendre un ou plusieurs excipients ou véhicules inertes, c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

La quantité de composé de formule I selon l'invention présent dans la composition thérapeutique peut être modulée de façon à obtenir un taux circulant de principe actif nécessaire à l'obtention de l'effet thérapeutique désiré pour un patient particulier, une composition, un mode d'administration, et ce, sans toxicité pour le patient.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées, puis ces doses seront progressivement augmentées afin de mieux maîtriser l'apparition d'éventuels effets secondaires.

En général la dose journalière du composé sera la dose minimum pour obtenir l'effet thérapeutique. Cette dose dépendra des différents facteurs cités auparavant. Les doses seront en général comprises entre 0,001 à 100 mg par kilo par jour pour l'homme, et préférentiellement de 0,001 à 10 mg par kilo et par jour et encore plus avantageusement de 0,01 à 1 mg par kilo et par jour.

Si nécessaire, la dose journalière peut être administrée en deux, trois, quatre, cinq, six ou plus, prises par jour ou par sous-doses multiples administrées par intervalles appropriés pendant la journée.

Comme indiqué précédemment, la demanderesse, après de longues recherches, a mis en évidence pour les composés chimiques répondant à la formule I, ainsi que leurs sels d'addition avec les acides pharmaceutiquement acceptables, présentent de remarquables propriétés thérapeutiques, notamment dans le traitement ou la prévention des pathologies neurodégénératives.

Ces propriétés sont illustrées par ailleurs dans la partie expérimentale. Elles justifient l'utilisation des composés ci-dessus décrits ainsi que de leurs sels d'addition avec les acides pharmaceutiquement acceptables à titre de médicament.

En conséquence et selon encore un autre aspect, l'invention a aussi pour objet l'utilisation des composés de formule I 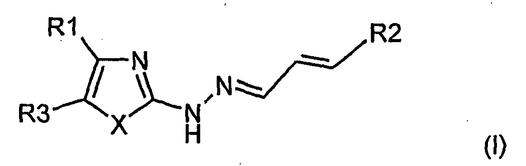 dans laquelle X, R1, R2 et R3 peuvent avoir les significations précédemment décrites, y compris la
- 3-phényl-2-propénal-[4-(4-nitrophényl)-2-thiazolyl] hydrazone ;
- 3-phényl-2-propénal-[4-(4-méthoxyphényl)-2-thiazolyl] hydrazone ;
- 3-phényl-2-propénal-[4-(4-fluorophényl)-2-thiazolyl] hydrazone;
- 3-phényl-2-propénal-[4-(4-bromophényl)-2-thiazolyl] hydrazone ;
- 3-phényl-2-propénal-[4-(4-chlorophényl)-2-thiazolyl] hydrazone ;
- 3-phényl-2-propénal-[4-(4-méthylphényl)-2-thiazolyl] hydrazone ;
- 3-phényl-2-propénal- [4-(1-naphthalènyl)-2-thiazolyl] hydrazone ;
- 3-phényl-2-propénal-(5-méthyl-4-phényl-2-thiazolyl) hydrazone ;
- 3-phényl-2-propénal-[4-(2,4-dichlorophényl)-2-thiazolyl] hydrazone ;
- 3-phényl-2-propénal-[4-(3-nitrophényl)-2-thiazolyl] hydrazone ;
- 3-phényl-2-propénal-[4-(1,1-diméthyléthyl)-2-thiazolyl] hydrazone ;
- 3-phényl-2-propénal-[4-(4-chlorophényl)-5-phényl-2-thiazolyl] hydrazone ;
- 2-buténal-[4-(4-nitrophényl)-2-thiazolyl] hydrazone ;
- 3-phényl-2-propénal-[5-(4-chlorophényl)-4-phényl-2-thiazolyl] hydrazone ;
- 3-(4-nitrophényl)-2-propénal-(4,5-diphényl-2-thiazolyl) hydrazone ;
- 3-phényl-2-propénal-(4,5-diphényl-2-thiazolyl) hydrazone ;
- acide 2-[[3-(3-nitrophényl)-2-propénylidène]hydrazino]-4-thiazole carboxylique
- acide 2-[[3-(2-nitrophényl)-2-propénylidène]hydrazino]-4-thiazole carboxylique
- 3-phényl-2-propénal-[4-chlorométhyl)-2-thiazolyl] hydrazone ;
- cinnamaldéhyde-(4-phényl-2-thiazolyl) hydrazone ;
- crotonaldéhyde-(4-phényl-2-thiazolyl) hydrazone ;
- 2-furanacroléine-(4-méthyl-2-thiazolyl) hydrazone ;
à titre de médicament.

Selon l'invention,
- la 3-(2-fluorophényl)-2-propénal-[4-(4-chlorophényl-2-thiazolyl) hydrazone ;
- la 3-(3-nitrophényl)-2-propénal-[4-(4-chlorophényl-2-thiazolyl) hydrazone ;
- la 3-(2-méthoxyphényl)-2-propénal-[4-(4-chlorophényl-2-thiazolyl) hydrazone ;
- la 3-(3-méthoxyphényl)-2-propénal-[4-(4-chlorophényl-2-thiazolyl) hydrazone ;
- la 3-phényl-2-propénal-[4-(3-hydroxyphényl-2-thiazolyl)-hydrazone ;
- la 3-pyridin-4-yle-2-propénal-[4-(4-chlorophényl-2-thiazolyl) hydrazone ;
- la 3-(2-méthylphényl)-2-propénal-[4-(4-chlorophényl-2-thiaxolyl) hydrazone ;
- la 3-(3-cyanophényl)-2-propénal-[4-(4-chlorophényl-2-thiazolyl) hydrazone ;
- la 3-(2-chlorophényl)-2-propénal-(4-(4-chlorophényl-2-thiazolyl) hydrazone ;
- la 3-(3-aminophényl)-2-propénal-[4-(4-chlorophényl-2-thiazolyl) hydrazone ;
- la 3-(3-nitrophényl)-2-propénal-[4-(4-trifluorométhylphényl-2-thiazolyl) hydrazone ;
- le 3-((E)-3-{[4-(4-chlorophényl)-thiazol-2-yl]-hydrazono}-propényl)-phénol ;
- la 3-pyridin-4-yl-2-propénal-[4-(4-trifluorométhylphényl-2-thiazolyl) hydrazone ;
- la 3-(3-aminophényl)-2-propénal-[4-(4-trifluorométhylphényl-2-thiazolyl) hydrazone ;
- la 3-(3-méthanesulfonylphényl)-2-propénal-[4-(4-chlorophényl-2-thiazolyl) hydrazone ;
- la 3-(3-cyanophényl)-2-propénal-[4-(4-hydroxyphényl-2-thiazolyl) hydrazone;
- la 3-(3-benzo[1,3]dioxol-5-yl)-2-propénal-[4-(4-trifluorométhylphényl-2-thiazolyl) hydrazone ;
- la 3-phényl-2-propénal-[4-(4-chloro-2-méthoxyphényl-2-thiazolyl) hydrazone ;
- la 1-[3-((E)-3-{[4-(4-trifluorométhylphényl)-thiazol-2-yl]-hydrazono}-propényl)-phényl]-éthanone ;
- la 3-pyridin-2-yl-2-propénal-[4-(4-chlorophényl-2-thiazolyl) hydrazone ;
- la 3-pyridin-2-yl-2-propénal-[4-(4-trifluorométhylphényl-2-thiazolyl) hydrazone ;
- le 3-méthyl-4-(2-{N'-[(E)-3-phénylprop-2-èn-(E)-ylidène]-hydrazino}-thiazol-4-yl)-phénol ;
- le [3-((E)-3-{[4-(4-triftuorométhylphényl)-thiazo)-2-y)]-hydrazono}-propènyl)-phényl]-méthanol ;
- la 3-(4-fluorophényl)-2-propénal-[4-(4-trifluorométhylphényl-2-thiazolyl) hydrazone ;
- la 3-((E)-3-{[4-(4-trifluorométhylphényl)-thiazol-2-yl]-hydrazono}-propènyl)-benzamide ;
et tout particulièrement la 3-phényl-2-propénal-[4-(4-trifluorométhylphényl)-2-thiazolyl]hydrazone ; le 3-((E)-3-{[4-(4-trifluorométhylphényl)-thiazol-2-yl]-hydrazono}-propényl)-phénol ; la 3-(3-méthanesulfonylphényl)-2-propénal-[4-(4-trifluorométhylphényl-2-thiazolyl)hydrazone ; et la 3-(3-cyanophényl)-2-propénal-[4-(4-trifluorométhylphényl-2-thiazolyl)hydrazone sont préférentiellement utilisés à titre de médicament.

Selon encore un autre aspect, l'invention a pour objet l'utilisation des composés de formule I, y compris les composés
- 3-phényl-2-propénal-[4-(4-nitrophényl)-2-thiazolyl] hydrazone ;
- 3-phényl-2-propénal-[4-(4-méthoxyphényl)-2-thiazolyl] hydrazone ;
- 3-phényl-2-propénal-[4-(4-fluorophényl)-2-thiazolyl] hydrazone ;
- 3-phényl-2-propénal-[4-(4-bromophényl)-2-thiazolyl] hydrazone ;
- 3-phényl-2-propénal-[4-(4-chlorophényl)-2-thiazolyl] hydrazone ;
- 3-phényl-2-propénal-[4-(4-méthylphényl)-2-thiazolyl] hydrazone ;
- 3-phényl-2-propénal- [4-(1-naphthalènyl)-2-thiazolyl] hydrazone ;
- 3-phényl-2-propénal-(5-méthyl-4-phényl-2-thiazolyl) hydrazone ;
- 3-phényl-2-propénal-[4-(2,4-dichlorophényl)-2-thiazolyl] hydrazone ;
- 3-phényl-2-propénal-[4-(3-nitrophényl)-2-thiazolyl] hydrazone ;
- 3-phényl-2-propénal-[4-(1,1-diméthyléthyl)-2-thiazolyl] hydrazone ;
- 3-phényl-2-propénal-[4-(4-chlorophényl)-5-phényl-2-thiazolyl] hydrazone ;
- 2-buténal-[4-(4-nitrophényl)-2-thiazolyl] hydrazone ;
- 3-phényl-2-propénal-[5-(4-chlorophényl)-4-phényl-2-thiazolyl] hydrazone ;
- 3-(4-nitrophényl)-2-propénal-(4,5-diphényl-2-thiazolyl) hydrazone ;
- 3-phényl-2-propénal-(4,5-diphényl-2-thiazolyl) hydrazone ;
- acide 2-[[3-(3-nitrophényl)-2-propénylidène]hydrazino]-4-thiazole carboxylique
- acide 2-[[3-(2-nitrophényl)-2-propénylidène]hydrazino]-4-thiazole carboxylique
- 3-phényl-2-propénal-(4-chlorométhyl)-2-thiazolyl] hydrazone ;
- cinnamaldéhyde-(4-phényl-2-thiazolyl) hydrazone ;
- crotonaldéhyde-(4-phényl-2-thiazolyl) hydrazone ;
- 2-furanacroléine-(4-méthyl-2-thiazolyl) hydrazone ;
- cinnamaldéhyde-(4-méthyl-2-thiazolyl) hydrazone ;
ou de leurs sels d'addition avec les acides pharmaceutiquement acceptables, dans la préparation d'une composition pharmaceutique destinée au traitement des pathologies neurodégénératives.

Sous un autre aspect, l'invention a pour objet l'utilisation des composés de formule I, y compris les composés
- 3-phényl-2-propénal-[4-(4-nitrophényl)-2-thiazolyl] hydrazone ;
- 3-phényl-2-propénal-[4-(4-méthoxyphényl)-2-thiazolyl] hydrazone ;
- 3-phényl-2-propénal-[4-(4-fluorophényl)-2-thiazolyl] hydrazone ;
- 3-phényl-2-propénal-[4-(4-bromophényl)-2-thiazolyl] hydrazone ;
- 3-phényl-2-propénal-[4-(4-chlorophényl)-2-thiazolyl] hydrazone ;
- 3-phényl-2-propénal-[4-(4-méthylphényl)-2-thiazolyl] hydrazone ;
- 3-phényl-2-propénal- [4-(1-naphthalènyl)-2-thiazolyl] hydrazone ;
- 3-phényl-2-propénal-(5-méthyl-4-phényl-2-thiazolyl) hydrazone ;
- 3-phényl-2-propénal-[4-(2,4-dichlorophényl)-2-thiazolyl] hydrazone ;
- 3-phényl-2-propénal-[4-(3-nitrophényl)-2-thiazolyl] hydrazone ;
- 3-phényl-2-propénal-[4-(1,1-diméthyléthyl)-2-thiazolyl] hydrazone ;
- 3-phényl-2-propénal-[4-(4-chlorophényl)-5-phényl-2-thiazolyl] hydrazone ;
- 2-buténal-[4-(4-nitrophényl)-2-thiazolyl] hydrazone ;
- 3-phényl-2-propénal-[5-(4-chlorophényl)-4-phényl-2-thiazolyl] hydrazone ;
- 3-(4-nitrophényl)-2-propénal-(4,5-diphényl-2-thiazolyl) hydrazone ;
- 3-phényl-2-propénal-(4,5-diphényl-2-thiazolyl) hydrazone ;
- acide 2-[[3-(3-nitrophényl)-2-propénylidène]hydrazino]-4-thiazole carboxylique
- acide 2-[[3-(2-nitrophényl)-2-propénylidène]hydrazino]-4-thiazole carboxylique
- 3-phényl-2-propénal-[4-chlorométhyl)-2-thiazolyl] hydrazone ;
- cinnamaldéhyde-(4-phényl-2-thiazolyl) hydrazone ;
- crotonaldéhyde-(4-phényl-2-thiazolyl) hydrazone ;
- 2-furanacroléine-(4-méthyl-2-thiazolyl) hydrazone ;
- cinnamaldéhyde-(4-méthyl-2-thiazolyl) hydrazone ;
ou de leurs sels d'addition avec les acides pharmaceutiquement acceptables, dans la préparation d'une composition pharmaceutique destinée au traitement des maladies chroniques neurodégénératives, particulièrement des maladies chroniques neurodégénératives à polyglutanine, héréditaires ou sporadiques, très particulièrement la maladie de Huntington (HD).

Ainsi les composés selon l'invention peuvent être utilisés dans la préparation de compositions pharmaceutiques destinées au traitement de la maladie d'Alzheimer (AD), la maladie de Parkinson (PD), l'atrophie musculaire spino-bulbaire ou maladie de Kennedy, les démences à corps de Lewy, les ataxies spino-cérébelleuses, la sclérose latérale amyotrophique (ALS), les amyotrophies spinales (SMA), la maladie de Creutzfeldt-Jakob, la sclérose en plaque (MS), l'adrénoleucodystrophie, l'épilepsie, les démences, la schizophrénie, la DRPLA, les syndromes neurologiques associés au syndrome d'immuno-déficience acquis (SIDA), les lésions neuronales liées au vieillissement, les neuropathies périphériques héréditaires ou lésionnelles, comme les maladies de Fabry, de Charcot-Marie-Tooth, de Krabbe, les leucodystrophies, les neuropathies diabétiques et celles induites par les traitements anti-cancéreux, les traumatismes du cerveau, des nerfs périphériques ou de la moëlle épinière, les ischémies du cerveau ou de la moëlle épinière suite à un accident cérébro-vasculaire, ou induites par un manque d'irrigation sanguine, les dégénérescences, héréditaires, lésionnelles ou liées au vieillissement des neurones sensoriels de la vision, comme les dégénérescences maculaires, les rétinites pigmentaires, ou les dégénérescences du nerf optique induites par les glaucomes, les dégénérescences, héréditaires, traumatiques ou liées au vieillissement des neurones sensoriels de l'ouïe entraînant une diminution ou une perte de l'audition.

D'autres aspects et avantages de la présente invention apparaîtront à la lecture des exemples qui suivent, qui doivent être considérés comme illustratifs et non limitatifs.

### Exemple 1 : synthèse de la 3-(2-fluorophényl)-2-propénal-[4-(4-chlorophényl-2-thiazolyl) hydrazone

### Etape 1A : synthèse du 2-fluorocinnamaldéhyde thiosemicarbazone :

390 mg (2,6 mmoles) de 2-fluorocinnamaldéhyde (Org. Lett. 5(5), 777-780, 2003) sont solubilisés dans 10 ml de méthanol anhydre. 236 mg (2,6 mmoles) de thiosemicarbazide sont ajoutés. Le milieu réactionnel est chauffé à reflux pendant 90 minutes.

La solution est refroidie à température ambiante, le précipité formé est filtré.

On obtient ainsi 250 mg de 2-fluorocinnamaldéhyde thiosemicarbazone (rendement : 43%).
Analyse :
RANZ ¹H : DMSO δ 11,45 (s, 1H), 8,21 (s, 1H), 7,90 (m, 1H), 7,67 (m, 2H), 7,36 (m, 1H), 7,25 (m, 2H), 7,02 (m, 2H).
LCMS [M+H]⁺= 225

### Etape 1B : synthèse de la 3-(2-fluorophényl)-2-propénal-[4-(4-chlorophényl-2-thiazolyl) hydrazone :

130 mg (0,5 mmoles) de 2-bromo-4'-chloroacétophénone et 124 mg de 2-fruorocinnamaldéhyde thiosemicarbazone préparé à l'étape 1A sont solubilisés dans 5 ml d'éthanol absolu. Le milieu réactionnel est chauffé à 60°C pendant 1 nuit.

La solution est refroidie à température ambiante. Le produit obtenu après filtration est purifié par flash chromatographie. On obtient ainsi 90 mg de 3-(2-fluorophényl)-2-propénal-[4-(4-chlorophényl-2-thiazolyl) hydrazone ; (rendements 50%).
Analyse :
RMN ¹H : DMSO δ 12,2 (s, 1H), 7,83 (m, 3H), 7,81 (t, 1H), 7,47 (d, 2H), 7,40 (s, 1H), 7,36 (m, 1H), 7,23 (m, 2H), 7,04 (s, 2H)
LCMS [M+H]⁺ 358/360

### Exemple 2 : synthèse de la 3-(3-nitrophényl)-2-propénal-[4-(4-chlorophényl-2-thiazolyl) hydrazone

### Etape 2A : synthèse de la-3-nitrocinnamaldéhyde thiosemicarbazone :

Le composé est préparé selon la méthode décrite à l'exemple 1A à partir de 3-nitrocinnamaldéhyde (2,2 mmoles) (Org. Lett. 5(5), 777-780, 2003) et de thiosemicarbazide (2,2 mmoles). Rendement 88%.
Analyse :
RMN ¹H δ 11,45 (s, 1H), 8,36 (d, 1H), 8,28 (m, 1H), 8,14 (m, 1H), 8,01 (m, 1H), 7,92 (m, 1H), 7,67 (m, 2H), 7,22 (m, 1H), 7,04 (m, 1H).
LCMS [M+1]⁺ =251

### Etape 2B : synthèse de la 3-(3-nitrophényl)-2-propénal-[4-(4-chlorophényl-2-thiazolyl) hydrazone;

Le composé est préparé selon la méthode décrite à l'exemple 1B à partir de 3-nitrocinnamaldéhyde thiosemicarbazone (0,5 mmoles) préparé à l'étape 2A et de 2-bromo-4'-chloroacétophénone (0,5 mmoles). Rendement 53%.
Analyse :
RMN ¹H : DMSO δ 12,26 (s, 1H), 8,40 (s, 1H), 8,11 (d, 2H), 7,91 (m, 3H), 7,66 (t, 1H), 7,47 (d, 2H), 7,42 (s, 1H), 7,23 (m, 1H), 7,12 (m, 1H).
LCMS [M+H]⁺ = 384/386

### Exemple 3 : synthèse de la 3-(2-méthoxyphényl)-2-propénal-[4-(4-chlorophényl-2-thiazolyl) hydrazone

### Etape 3A : synthèse de la 2-méthoxycinnamaldéhyde thiosemicarbazone :

Le composé est préparé selon la méthode décrite à l'exemple 1A à partir de 2-méthoxycinnamaldéhyde (6 mmoles) et de thiosemicarbazide (6 mmoles). Rendement 45 %.
Analyse :
RMN ¹H : DMSO δ 11,32 (s, 1H), 8,14 (t, 1H), 7,87 (d, 1H), 7,60 (m, 1H), 7,52 (d, 1H), 7,32 (m, 1H), 7,12 (t, 2H), 6,95 (m, 3H), 3,85 (s, 3H).
LCMS [M+1]⁺ = 237

### Etape 3B : synthèse de la 3-(2-méthoxyphényl)-2-propénal-[4-(4-chlorophényl-2-thiazolyl) hydrazone :

Le composé est préparé selon la méthode décrite à l'exemple 1B à partir de 2-méthoxycinnamaldéhyde thiosemicarbazone (0,5 mmoles) préparé à l'étape 3A et de 2-bromo-4'-chloroacétophénone (0,5 mmoles). Rendement 38%.
Analyse :
RMN ¹H : DMSO δ 12,26 (s, 1H), 7,73 (d, 2H), 7,66 (m, 1H), 7,52 (d, 1H), 7,41 (m, 1H), 7,08 (s, 1H), 7,00 (m, 2H), 6,92 (m, 1H), 6,84 (s, 1H), 3.92 (s, 3H).
LCMS [M+H]⁺ = 370/372

### Exemple 4 : synthèse de la 3-phényl-2-propénal-[4-(4-trifluorométhylphényl)-2-thiazolyl]hydrazone

Le composé est préparé selon la méthode décrite à l'exemple 1B à partir de cinnamaldéhyde thiosemicarbazone (0,5 mmoles) (Eur. J. Med. Chem. 25(7), 581-588, 1990) et de 2-bromo-4'-trifluorométhylacétophénone (0,5 mmoles) (Tet., 59(8) 1317-1325, 2003). Rendement 42%.
Analyse :
RMN ¹H : DMSO δ 12,19 (s, 1H), 8,06 (d, 2H), 7,88 (d, 1H), 7,77 (d, 2H), 7,61 (d, 2H), 7,57 (s, 1H), 7,39 (m, 2H), 7,31 (m, 1H), 6,98 (m, 2H).
LCMS [M+H]⁺ = 374

### Exemple 5 : synthèse de la 3-phényl-2-propénal-[4-(4-chlorophényl-5-méthyl-2-thiazolyl) hydrazone

Le composé est préparé selon la méthode décrite à l'exemple 1B à partir de cinnamaldéhyde thiosemicarbazone (0,12 mmoles) (Eur. J. Med. Chem. 25(7), 581-588, 1990) et de 2-bromo-4'-chloropropiophénone (0,12 mmoles) (J. Org. Chem. 68(4) 1594-1596, 2003). Rendement 58%.
Analyse :
RMN ¹H : DMSO δ 7,95 (d, 1H), 7,52 (m, 6H), 7,42 (m, 3H), 6,94 (m, 2H), 2,46 (s, 3H).
LCMS [M+H]⁺ = 354/356

### Exemple 6 : synthèse de la 3-(3-méthoxyohényl)-2-propénal-[4-(4-chlorophényl-2-thiazolyl) hydrazone

### Etape 6A : synthèse de la 3-méthoxycinnamaldéhyde thiosemicarbazone :

Le composé est préparé selon la méthode décrite à l'exemple 1A à partir de 3-méthoxycinnamaldéhyde (2,47 mmoles) et de thiosemicarbazide (2,47 mmoles). Rendement 52%.
Analyse :
LCMS [M+1]⁺ = 237

### Etape 6B : synthèse de la 3-(3-méthoxyphényl)-2-propénal-[4-(4-chlorophényl-2-thiazolyl) hydrazone :

Le composé est préparé selon la méthode décrite à l'exemple 1B à partir de 3-méthoxycinnamaldéhyde thiosemicarbazone (0,52 mmoles) préparé à l'étape 6A et de 2-bromo-4'-chloroacétophénone (0,43 mmoles). Rendement 25%.
Analyse :
RMN ¹H : DMSO δ 12,12 (s, 1H), 7,86 (m, 3H), 7,47 (d, 2H), 7,39 (s, 1H), 7,28 (t, 1H), 7,17 (m, 2H), 7,01 (m, 1H), 6,94 (s, 1H), 6,87 (m, 1H), 3,82 (s, 3H).
LCMS [M+H]⁺ = 370/372

### Exemple 7 : synthèse de la 3-phényl-2-propénal-[4-(3-hydroxyphényl-2-thiazolyl)hydrazone

Le composé est préparé selon la méthode décrite à l'exemple 1B à partir de cinnamaldéhyde thiosemicarbazone (0,28 mmoles) (Eur. J. Med. Chem. 25(7), 581-588, 1990) et de 2-bromo-4'-hydroxyacétophénone (0,46 mmoles). Rendement 28%.
RMN ¹H : DMSO δ 12,07 (s, 1H), 9,45 (s, 1H), 7,87 (d, 1H), 7,61 (d, 2H), 7,38 (m, 2H), 7,32 (m, 3H), 7,25 (m, 2H); 6,97 (m, 2H), 6,71 (d, 1H).
LCMS [M+H]⁺ = 322

### Exemple 8 : synthèse de la 3-pyridin-4-yl-2-propénal-[4-(4-chlorophényl-2-thiazolyl) hydrazone

Le composé est préparé selon la méthode décrite à l'exemple 1B à partir de 2-[3-(4-pyridinyl)-2-propénylidène]-hydrazinecarbothioamide (0,097 mmoles) (Eur. J. Med. Chem., 1995, 30(12), 983-988) et de 2-bromo-4'-chloroacétophénone (0,097 mmoles) Rendement 45%.
RMN ¹H : DMSO δ 12,30 (s, 1H), 8,54 (d, 2H), 7,86 (m, 3H), 7,56 (m, 2H), 7,47. (d, 2H), 7,42 (s, 1H), 7,35 (m, 1H), 6,94 (d, 1H).
LCMS [M+H]⁺ = 341/343

### Exemple 9 : synthèse de la 3-(2-méthylphényl)-2-propénal-[4-(4-chlorophényl-2-thiazolyl) hydrazone

### Etape 9A : synthèse du 2-méthylcinnamaldéhyde thiosemicarbazone :

Le composé est préparé selon la méthode décrite à l'exemple 1A à partir de 2-méthylcinnamaldéhyde (0,27 mmoles) et de thiosemicarbazide (0,27 mmoles). Rendement 42%.
RMN ¹H: DMSO δ 11,34 (s, 1H), 8,24 (t, 1H), 7,92 (d, 1H), 7,61 (m, 1H), 7,55 (m, 1H), 7,21 (m, 3H), 7,17 (m, 1H), 6,73 (m, 1H), 2,36 (s, 3H).
LCMS [M+1 ]⁺ = 220

### Etape 9B : synthèse de la 3-(2-méthylphényl)-2-propénal-[4-(4-chlorophényl-2-thiazolyl) hydrazone :

Le composé est préparé selon la méthode décrite à l'exemple 1B à partir de 2-méthylcinnamaldéhyde thiosemicarbazone (0,07 mmoles) préparé à l'étape 9A et de 2-bromo-4'-chloroacétophénone (0,07 mmoles). Rendement 41%.
RMN ¹H : DMS δ 12,12 (s, 1H), 7,91 (d, 1H), 7,86 (d, 2H), 7,70 (m, 1H), 7,46 (d, 2H), 7,38 (s, 1H), 7,18 (m, 3H), 7,13 (s, 1H), 6,90 (m, 1H), 2,37 (s, 3H).
LCMS [M+H]⁺ = 354/356

### Exemple 10 : synthèse de la 3-(3-cyanophényl)-2-propénal-[4-(4-chlorophényl-2-thiazolyl) hydrazone ;

### Etape 10A : synthèse du 3-cyanocinnamaldéhyde thiosemicarbazone :

Le composé est préparé selon la méthode décrite à l'exemple 1A à partir de 3-cyanocinnamaldéhyde (2,54 mmoles) et de thiosemicarbazide (2,54 mmoles). Rendement 46%.
Analyse :
RMN ¹H : 11,45 (s, 1H), 8,26 (d, 1H), 8,05 (s, 1H), 7,89 (m, 2H), 7,77 (m, 1H), 7,58 (m, 2H), 7,07 (m, 2H).
LCMS [M+1]⁺ = 231

### Etape 10B : synthèse de la 3-(3-cyanophényl)-2-propénal-[4-(4-chlorophényl-2-thiazolyl) hydrazone :

Le composé est préparé selon la méthode décrite à l'exemple 1B à partir de 3-cyanocinnamaldéhyde thiosemicarbazone (0,22 mmoles) préparé à l'étape 10A et de 2-bromo-4'-chloroacétophénone (0,22 mmoles). Rendement 51%.
Analyse :
LCMS [M+H]⁺ = 365/367

### Exemple 11 : synthèse de la 3-(3-cyanophényl)-2-propénal-[4-(4-trifluorométhylphényl-2-thiazolyl)hydrazone

Le composé est préparé selon la méthode décrite à l'exemple 1B à partir de 3-cyanocinnamaldéhyde thiosemicarbazone (0,43 mmoles) préparé à l'étape 10A et de 2-bromo-4'-trifluorométhylacétophénone (0,43 mmoles) (Tet., 59(8) 1317-1325, 2003). Rendement 69%.
Analyse :
LCMS [M+H]⁺ = 399

### Exemple 12 synthèse de la 3-(2-chlorophényl)-2-propénal-[4-(4-chlorophényl-2-thiazoyl) hydrazone

### Etape 12A : synthèse du 2-chlorocinnamaldéhyde thiosemicarbazone :

Le composé est préparé selon la méthode décrite à l'exemple 1A à partir de 2-chlorocinnamaldéhyde (0,15 mmoles) et de thiosemicarbazide (0,15 mmoles). Rendement 55%.
LCMS [M+1]⁺ = 240/242

### Etape 12B : synthèse de la 3-(2-chlorophényl)-2-propénal-[4-(4-chlorophényl-2-thiazolyl) hydrazone :

Le composé est préparé selon la méthode décrite à l'exemple 1B à partir de 2-chlorocinnamaldéhyde thiosemicarbazone (0,08 mmoles) préparé à l'étape 12A et de 2-bromo-4'-chloroacétophénone (0,08 mmoles). Rendement 51%.
RMN ¹H : DMSO δ 12,25 (s, 1H), 7,95 (m, 2H), 7,86 (m, 2H), 7,48 (m, 3H), 7,42 (s, 1H), 7,35 (m, 2H), 7,18 (d, 1H), 7,13 (d, 1H).
LCMS [M+H]⁺= 375/377

### Exemple 13 : synthèse de la 3-(3-nitrophényl)-2-propénal-[4-(4-trifluorométhyphényl-2-thiazolyl) hydrazone

Le composé est préparé selon la méthode décrite à l'exemple 1B à partir de 3-nitrocinnamaldéhyde thiosemicarbazone (0;5 mmoles) préparé à l'étape 2A et de 2-bromo-4'-trifluorométhylacétophénone (0,5 mmoles). Rendement 52%.
Analyse :
LCMS [M+H]⁺ = 419

### Exemple 14 : synthèse de la 3-(3-aminophényl)-2-propénal-[4-(4-chlorophényl-2-thiazolyl) hydrazone

### Etape 14A : synthèse de la 3-aminocinnamaldéhyde thiosemicarbazone :

Une solution de 10 mL d'éthanol et de 2 mL de soude 2N est saturée en sulfure d'hydrogène 500 mg (2 mmoles) de 3-nitrocinnamaldéhyde thiosemicarbazone préparé à l'étape 2A sont ajoutés. La solution est chauffée 10 minutes à 50°C, puis 3 minutes à 75°C. La solution est refroidie dans un bain de glace. Les cristaux formés sont filtrés, lavés à l'éthanol. On obtient ainsi 299 mg de 3-aminocinnamaldéhyde thiosemicarbazone. Rendement 68%.
Analyse :
LCMS [M+1]⁺ = 221

### Etape 14B : synthèse de la 3-(3-aminophényl)-2-propénal-[4-(4-chlorophényl-2-thiazolyl) hydrazone :

Le composé est préparé selon la méthode décrite à l'exemple 1B à partir de 3-aminocinnamaldéhyde thiosemicarbazone (0,5 mmoles) préparé à l'étape 14A et de 2-bromo-4'-chloroacétophénone (0,5 mmoles). Rendement 53%.
Analyse :
RMN ¹H: DMSO δ 7,73 (d, 2H), 7,46 (d, 1H), 7,40 (d, 2H), 7,16 (t, 1H), 6,8 (m, 3H), 6,67 (m, 1H), 6,52 (d, 1H), 6,48 (d, 1H).
LCMS [M+H]⁺ = 355/357

### Exemple 15 : synthèse du 3-((E)-3-{[4-(4-chlorophényl)-thiazol-2-yl]-hydrazono}-propényl)-phénol

### Etape 15A : synthèse de la 3-hydroxycinnamaldéhyde thiosemicarbazone :

Le composé est préparé selon la méthode décrite à l'exemple 1A à partir de 3-hydroxycinnamaldéhyde (6 mmoles) et de thiosemicarbazide (6 mmoles). Rendement 28 %.
Analyse :
LCMS [M+1]⁺ = 222

### Etape 15B : synthèse du 3-((E)-3-{[4-(4-chlorophényl)-thiazol-2-yl]-hdrazono}-propényl)-phénol :

Le composé est préparé selon la méthode décrite à l'exemple 1B à partir de 3-hydroxycinnamaldéhyde thiosemicarbazone (0,5 mmoles) préparé à l'étape 15A et de 2-bromo-4'-chloroacétophénone (0,5 mmoles). Rendement 39%.
Analyse :
RMN ¹H : DMSO δ 12,10 (s, 1H), 9,47 (s, 1H), 7,85 (d, 3H), 7,46 (d, 2H), 7,38 (s, 1 H), 7,16 (t, 1 H), 7,03 (d, 1 H), 6,92 (m, 1 H), 6,85 (m, 3H), 6,70 (d, 1H).
LCMS [M+H]⁺ = 356/358

### Exemple 16 : synthèse de la 3-pyridin-4-yl-2-propénal-[4-(4-trifluorométhylphényl-2-thiazolyl) hydrazone

Le composé est préparé selon la méthode décrite à l'exemple 1B à partir de 2-[3-(4-pyridinyl)-2-propénylidène]-hydrazinecarbothioamide (0,097 mmoles) (Eur. J. Med. Chem., 1995, 30(12), 983-988) et de 2-bromo-4'-trifluorométhylacétophénone (0,097 mmoles) Rendement 45%.
RMN ¹H : DMSO δ 12,36 (s, 1H), 8,54 (d, 2H), 8,06 (m, 2H), 7,90 (d, 1H), 7,78 (d, 2H), 7,57 (m, 3H), 7,25 (m, 1 H), 6,94 (d, 1 H).
LCMS [M+H]⁺= 375

### Exemple 17 : synthèse de la 3-(3-aminophényl)-2-propénal-[4-(4-trifluorométhylphényl-2-thiazolyl) hydrazone

Le composé est préparé selon la méthode décrite à l'exemple 1B à partir de 3-aminocinnamaldéhyde thiosemicarbazone (0,5 mmoles) préparé à l'étape 14A et de 2-bromo-4'-trifluorométhylacétophénone (0,5 mmoles). Rendement 58%.
Analyse :
LCMS [M+H]⁺ = 389

### Exemple 18 : synthèse de la 3-(3-méthanesulfonylphényl)-2-propénal-[4-(4-chlorophényl-2-thiazolyl) hydrazone

### Etape 18A : synthèse de la 3-méthylsulfonylcinnamaldéhyde thiosemicarbazone :

Le composé est préparé selon la méthode décrite à l'exemple 1A à partir de 3-méthylsulfonylcinnamaldéhyde (3,8 mmoles) et de thiosemicarbazide (3,8 mmoles). Rendement 70%.
Analyse :
LCMS [M+1]⁺ = 284

### Etape 18B : synthèse de la 3-(3-méthanesulfonylphényl)-2-propénal-[4-(4-chlorophényl-2-thiazolyl)hydrazone :

Le composé est préparé selon la méthode décrite à l'exemple 1B à partir de 4-méthylsulfonylcinnamaldéhyde thiosemicarbazone (2,68 mmoles) préparé à l'étape 18A et de 2-bromo-4'-chloroacétophénone (2,68 mmoles). Rendement 60%.
Analyse :
RMN ¹H : DMSO δ 12,05 (s, 1H), 8,21 (s, 1H), 7,99 (m, 1H), 7,88 (m, 3H), 7,78 (m, 1H), 7,52 (m, 1H), 7,41 (m, 2H), 7,30 (m, 1H), 7,18 (m, 1H), 7,01 (m, 1 H), 3,34 (s, 3H).
LCMS [M+H]⁺ = 418/420

### Exemple 19 : synthèse de la 3-(3-cyanophényl)-2-propénal-[4-(3-hydroxyphényl-2-thiazolyl) hydrazone

Le composé est préparé selon la méthode décrite à l'exemple 1B à partir de 3-cyanocinnamaldéhyde thiosemicarbazone (0,22 mmoles) préparé à l'étape 10A et de 2-bromo-3'-hydroxyacétophénone (0,22 mmoles). Rendement 51 %.
Analyse :
RMN ¹H : DMSO δ 12, 10 (s, 1H), 9,45 (s, 1H), 8,11 (s, 1H), 7,90 (d, 1H), 7,78 (d, 1 H), 7,67 (m, 1 H), 7,56 (m, 1 H), 7,25 (m, 3H), 7,18 (t, 2H), 6,98 (d, 1 H), 6,68 (d, 1H).
LCMS [M+H]⁺ = 347

### Exemple 20 : synthèse du 3-((E)-3-{[4-(4-trifluorométhylphényl)-thiazol-2-yl]-hydrazono}-propényl)-phénol

Le composé est préparé selon la méthode décrite à l'exemple 1B à partir de 3-hydroxycinnamaldéhyde thiosemicarbazone (0,5 mmoles) préparé à l'étape 15A et de 2-bromo-4'-trifluorométhylacétophénone (0,5 mmoles). Rendement 31%.
Analyse :
RMN ¹H : DMSO δ 12,15 (s, 1H), 9,48 (s, 1H), 8,06 (d, 2H), 7,85 (d, 1H), 7,76 (d, 2H), 7,56 (s, 1H), 7,16 (t, 1 H), 7,04 (d, 1 H), 6,92 (m, 1 H), 6,72 (m, 2H), 6,70 (d, 1H).
LCMS [M+H]⁺ = 390

### Exemple 21 : synthèse de 3-(3-benzo[1,3]dioxol-5-yl)-2-propénal-[4-(4-trifluorométhylphényl-2-thiazolyl)hydrazone

### Etape 21A : synthèse du (3-benzo[1,3]dioxol-5-yl)-propénal thiosemicarbazone :

Le composé est préparé selon la méthode décrite à l'exemple 1A à partir de 2-(3-benzo[1,3]dioxol-5-yl)-propénal (0,15 mmoles) et de thiosemicarbazide (0,15 mmoles). Rendement 51%.
LCMS [M+1]⁺ = 250

### Etape 21B : synthèse de la 3-(3-benzo[1,3]dioxol-5-yl)-2-propénal-[4-(4-trifluorométhylphényl-2-thiazolyl)hydrazone :

Le composé est préparé selon la méthode décrite à l'exemple 1B à partir de du (3-benzo[1,3]dioxol-5-yl)-propénal thiosemicarbazone (0,08 mmoles) préparé à l'étape 21A et de 2-bromo-4'-trifluorométhylacétophénone (0,08 mmoles). Rendement 51%.
RMN ¹H : DMSO δ 12,20 (s, 1H), 8,06 (m, 2H), 7,76 (m, 2H), 7,68 (m, 2H), 7,54 (s, 1 H), 7,32 (s, 1 H), 6,92 (d, 1 H), 6,87 (m, 2H), 6,04 (s, 2H).
LCMS [M+H]⁺ = 418

### Exemple 22 : synthèse de la 3-phényl-2-propénal-[4-(4-chloro-2-méthoxyphényl-2-thiazolyl) hydrazone

Le composé est préparé selon la méthode décrite à l'exemple 1B à partir de cinnamaldéhyde thiosemicarbazone (0,28 mmoles) (Eur. J. Med. Chem. 25(7), 581-588, 1990) et de 2-bromo-4'-chloro-2'-méthoxyacétophénone (0,28 mmoles). Rendement 36%.
RMN ¹H : DMSO δ 12,06 (s, 1 H), 8,06 (d, 1 H), 7,87 (d, 1 H), 7,61 (d, 2H), 7,38 (m, 3H), 7,28 (m, 1 H), 7,17 (s, 1 H), 7,07 (m, 1 H), 6,98 (m, 2H), 3,94 (s, 3H).
LCMS [M+H]⁺ = 370/372

### Exemple 23 : synthèse de 3-((E)-3-{[4-(4-trifluorométhylphényl)-thiazol-2-yl]-hydrazono}-propényl)-benzamide

### Etape 23A : synthèse du 3-((E)-3-thiosemicarbazone-propényl)-benzamide :

Le composé est préparé selon la méthode décrite à l'exemple 1A à partir de 3-((E)-oxopropényl)-benzamide (0,15 mmoles) et de thiosemicarbazide (0,15 mmoles). Rendement 41%.
LCMS [M+1]⁺ = 249

### Etape 23B : synthèse du 3-((E)-3-{[4-(4-trifluorométhylphényl)-thiazol-2-yl]-hydrazono}-propényl)-benzamide :

Le composé est préparé selon la méthode décrite à l'exemple 1B à partir de 3-((E)-3-thiosemicarbazone-propényl)-benzamide (0,08 mmoles) préparé à l'étape 23A et de 2-bromo-4'-trifluorométhylacétophénone (0,08 mmoles). Rendement 51%.
RMN ¹H : DMSO δ 12,22 (s, 1H), 8,15 (s, 1H), 8,06 (d, 3H), 7,90 (d, 1H), 7,78 (m, 3H), 7,70 (m, 1 H), 7,57 (s, 1 H), 7,46 (m, 2H), 7,14 (m, 1 H), 7,00 (d, 1H).
LCMS [M+H]⁺ = 417

### Exemple 24 : synthèse de la 3-(3-méthanesulfonylphényl)-2-propénal-[4-(4-trifluorométhylphényl-2-thiazolyl)hydrazone

Le composé est préparé selon la méthode décrite à l'exemple 1B à partir de 4-méthylsulfonylcinnamaldéhyde thiosemicarbazone (2,95 mmoles) préparé à l'étape 18A et de 2-bromo-4'-trifluorométhylacétophénone (2,95 mmoles). Rendement 73%.

Analyse :
RMN ¹H : DMSO δ 12,29 (s, 1 H), 8,14 (s, 1 H), 8,05 (d, 2H), 7,97 (m, 2H), 7,78 (m, 3H), 7,63 (m, 1H), 7,58 (m, 1H), 7,24 (m, 1 H), 7,09 (m, 1H), 3,31 (s, 3H).
LCMS [M+H]⁺ = 452

### Exemple 25 synthèse de la 3-pyridin-2-yl-2-propénal-[4-(4-chlorophényl-2-thiazolyl) hydrazone

### Etape 25A : synthèse du 2-13-(2-pyridinyl)-2-propénylidénel-hydrazine carbothioamide :

Le composé est préparé selon la méthode décrite à l'exemple 1A à partir de (E)-3-pyridin-2-yl-propénal (0,2 mmoles) et de thiosemicarbazide (0,2 mmoles). Rendement 43%.
LCMS [M+1]⁺ = 207

### Etape 25B : synthèse de la 3-pyridin-2-yl-2-propénal-[4-(4-chlorophényl-2-thiazolyl) hydrazone :

Le composé est préparé selon la méthode décrite à l'exemple 1B à partir de 2-[3-(2-pyridinyl)-2-propénylidène]-hydrazinecarbothioamide (0,097 mmoles) et de 2-bromo-4'-chloroacétophénone (0,097 mmoles) Rendement 41%.
RMN ¹H : DMSO δ 12,30 (s, 1 H), 8,58 (m, 1H), 7,90 (m, 1H), 7,87 (m, 2H), 7,78 (m, 1 H), 7,59 (d, 1 H), 7,46 (d, 2H), 7,41 (s, 1 H), 7,30 (m, 2H), 6,98 (d, 1 H).
LCMS [M+H]⁺ = 341/343

### Exemple 26 synthèse de la 3-pyridin-2-yl-2-propénal-[4-(4-trifluorométhylphényl-2-thiazolyl) hydrazone

Le composé est préparé selon la méthode décrite à l'exemple 1B à partir de 2-[3-(2-pyridinyl)-2-propénylidène]-hydrazinecarbothioamide préparé à l'étape 25A (0,11 mmoles) et de 2-bromo-4'-trifluorométhylacétophénone (0,11 mmoles) Rendement 46%.
RMN ¹H : DMSO δ 12,30 (s, 1H), 8,58 (m, 1H), 8,07 (d, 2H), 7,92 (d, 1H), 7,77 (m, 8H), 7,59 (m, 2H), 7,30 (m, 2H), 7,00 (d, 1H).
LCMS [M+H]⁺= 375

### Exemple 27 : synthèse du 3-méthyl-4-(2-{N'-[(E)-3-phénylprop-2-èn-(E)-lidène]-hydrazino}-thiazol-4-yl)-phénol

Le composé est préparé selon la méthode décrite à l'exemple 1 B à partir de cinnamaldéhyde thiosemicarbazone (0,28 mmoles) (Eur. J. Med. Chem. 25(7), 581-588, 1990) et de 2-bromo-4'-hydroxy-2'-méthylacétophénone (0,28 mmoles). Rendement 36%.
RMN ¹H : DMSO δ 11,85 (s, 1H), 9,42 (s, 1H), 7,82 (d, 1H), 7,64 (d, 2H), 7,36 (m, 3H), 7,31 (m, 1H), 6,92 (m, 2H), 6,66 (s, 1 H), 6,63 (m, 2H), 2,35 (s, 3H).
LCMS [M+H]⁺ = 336

### Exemple 28 : synthèse du [3-((E)-3-{[4-(4-trifluorométhylphényl)-thiazol-2-yl]-hydrazono}-propènyl)-phényl]-méthanol

### Etape 28A : synthèse de la 3-hydroxyméthvicinnamaldéhyde thiosemicarbazone:

Le composé est préparé selon la méthode décrite à l'exemple 1A à partir de 3-hydroxyméthylcinnamaldéhyde (3 mmoles) et de thiosemicarbazide (3 mmoles). Rendement 25 %.
Analyse :
LCMS [M+1]⁺ = 236

### Etape 28B : synthèse du [3-((E)-3-{[4-(4-trifluorométhylphényl)-thiazol-2-yl]-hydrazono}-propènyl-phényl]-méthanol:

Le composé est préparé selon la méthode décrite à l'exemple 1B à partir de 3-hydroxyméthylcinnamaldéhyde thiosemicarbazone (0,4 mmoles) préparé à l'étape 28A et de 2-bromo-4'-trifluorométhylacétophénone (0,4 mmoles). Rendement 37%.
Analyse:
RMN ¹H : DMSO δ 12,18 (s, 1H), 8,05 (m, 2H), 7,88 (m, 1H), 7,76 (d, 2H), 7,54 (m, 2H), 7,48 (m, 1 H), 7,33 (t, 1 H), 7,25 (m, 1 H), 6,96 (m, 2H), 5,21 (m, 1 H), 4,52 (m, 2H).
LCMS [M+H]⁺ = 404

### Exemple 29 synthèse de la 3-(4-fluorophényl)-2-propénal-[4-(4-trifluorométhylphényl-2-thiazolyl)hydrazone

### Etape 29A : synthèse de la 4-fluorocinnamaldéhyde thiosemicarbazone :

Le composé est préparé selon la méthode décrite à l'exemple 1A à partir de 4-fluorocinnamaldéhyde (1.5 mmoles) et de thiosemicarbazide (1.5 mmoles). Rendement 53 %.
Analyse :
LCMS [M+1]⁺ = 224

### Etape 29B : synthèse de la 3-(4-fluorophényl)-2-propénal-[4-(4-trifluorométhylphényl-2-thiazolyl)hydrazone ;

Le composé est préparé selon la méthode décrite à l'exemple 1 B à partir de 4-fluorocinnamaldéhyde thiosemicarbazone (0,3 mmoles) préparé à l'étape 29A et de 2-bromo-4'-trifluorométhylacétophénone (0,3 mmoles). Rendement 46%.
Analyse :
RMN ¹H : DMSO δ 12,18 (s, 1H), 8,05 (d, 2H), 7,86 (m, 1H), 7,77 (m, 2H), 7,68 (m, 2H), 7,56 (s, 1 H), 7,21 (t, 2H), 6,96 (m, 2H).
LCMS [M+H]⁺ = 392

### ETUDE PHARMACOLOGIQUE

Pour mettre en évidence les propriétés neuroprotectrices des composés de formule I selon l'invention, la demanderesse a étudié leur activité sur un modèle *in vitro* d'expression de la huntingtine mutée dans des neurones primaires isolés du striatum de rat. Ce modèle mime les conditions de dégénérescence de la maladie d'Huntington. Il permet donc d'identifier des molécules qui combinent plusieurs des mécanismes d'action décrits précédemment pour réduire le dysfonctionnement précédant la mort neuronale et promouvoir la survie des neurones dysfonctionnels.

### Exemple 30 : Evaluation de la protection conférée par les composés de formule I sur des neurones striataux dans un test de mort induite par la surexpression d'une forme mutée de la huntingtine en utilisant la GFP comme gène rapporteur

Toutes les techniques de biologie moléculaire utilisées dans cet exemple sont des techniques classiques du domaine, parfaitement connues de l'homme du métier et que l'on peut retrouver dans n'importe quel ouvrage relatif auxdites techniques.

Des cultures primaires de neurones striataux sont préparées comme décrit dans la littérature (Mao L. et al., Methods Mol. Med., 2003, 79 : 379-86).

Avant ensemencement, un vecteur d'expression contenant un élément promoteur suivi de l'ADN codant pour une forme tronquée de la huntingtine qui comprend les 480 premiers acides aminés et 68 codons CAG (Saudou et al., Cell, 1998, 95 :55-66), préalablement purifié, est introduit par électroporation d'après la procédure décrite par Raoul et al., (Neuron, 2002, 35 :1067-83).

Un second vecteur d'expression, également préalablement purifié, contenant l'ADN codant la protéine fluorescente verte (*green fluorescent protein* (GFP) (Columbia University) est également électroporé et sert de gène rapporteur.

Les cellules qui survivent à l'électroporation sont ensemencées à une densité de 4000 cellules par puits sur des plaques 96 puits. La culture se fait dans 200 µl de milieu Neurobasal (GIBCO) complémenté avec 1mM final de pyruvate et du B-27 1/100 (Beckton Dickinson). Les cellules sont maintenues en culture pendant 6 jours sans changer le milieu.

Les traitements avec les composés à tester se font juste après l'ensemencement à une concentration finale de 3 µM dans 0,5% de diméthylsulfoxyde (DMSO). Les contrôles positifs se font par adjonction de BDNF (Brain-Derived Neurotrophic Factor) à 5 ng/ml final (Tébu). Les contrôles négatifs ne reçoivent que 0,5% de DMSO.

La mort cellulaire est évaluée après les 6 jours par comptage du nombre de cellules vivantes exprimant la GFP.

L'activité des composés à tester a été évaluée par leur capacité à empêcher la mort des neurones striataux cultivés dans le milieu Neurobasal en comparaison avec la survie des neurones striataux en milieu supplémenté avec du BDNF.

### Résultats :

Les résultats obtenus sont présentés dans le tableau 1 récapitulatif situé ci-dessous.

Les résultats sont exprimés sous la forme d'un rapport évaluant la survie des cellules GFP-positives en présence du composé à tester. Il s'agit donc du nombre de cellules vivantes après traitement avec le composé à tester diminué du nombre de cellules vivantes après traitement au DMSO, rapporté (divisé par) au nombre de cellules survivantes après traitement par le BDNF, diminué du nombre de cellules vivantes après traitement au DMSO.

Ce rapport représente donc le pourcentage de survie dû au composé testé par rapport à la survie induite par le BDNF.

Dans ce test un composé est considéré comme actif quand ledit rapport est supérieur à 0,2 c'est-à-dire quand il présente une activité neuroprotectrice au moins égale à 20% de l'activité neuroprotectrice du BDNF.

**Tableau 1 : Tableau récapitulatif des activités pharmacologiques des produits de formule I**

| Composé de l'exemple n° | Concentration en µM | Rapport GFP |
|---|---|---|
| 1 | 1 | > 0,2 |
| 2 | 3 | > 0,2 |
| 3 | 3 | > 0,2 |
| 4 | 1 | > 0,2 |
| 6 | 10 | > 0,2 |
| 7 | 3 | > 0,2 |
| 8 | 10 | > 0,2 |
| 9 | 3 | > 0,2 |
| 10 | 1 | > 0,2 |
| 11 | 1 | > 0,2 |
| 12 | 10 | > 0,2 |
| 13 | 1 | > 0,2 |
| 14 | 1 | > 0,2 |
| 15 | 1 | > 0,2 |
| 16 | 3 | > 0,2 |
| 17 | 1 | > 0,2 |
| 18 | 1 | > 0,2 |
| 19 | 3 | > 0,2 |
| 20 | 0,3 | > 0,2 |
| 21 | 1 | > 0,2 |
| 22 | 3 | > 0,2 |
| 23 | 0,3 | > 0,2 |
| 24 | 0,3 | > 0,2 |
| 25 | 1 | > 0,2 |
| 26 | 0,3 | > 0,2 |
| 27 | 3 | > 0,2 |
| 28 | 0,3 | > 0,2 |
| 29 | 3 | > 0,2 |
| 31* | 3 | > 0,2 |
| 32** | 3 | > 0,2 |

| | | |
|---|---|---|
| 31* : 3-phényl-2-propénal-[4-(4-chlorophényl)-2-thiazolyl]hydrazone ; vendu par Chembridge. 32** : 3-phényl-2-propénal-[4-(4-méthylphényl)-2-thiazolyl]hydrazone ; vendu par Chembridge. | | |

De par leur effet neuroprotecteur, les composés de formule I selon l'invention se montrent donc comme de bons candidats médicaments neuroprotecteurs, utilisables dans le traitement des pathologies neurodégénératives.

## Revendications

1. Composés chimiques nouveaux répondant à la formule I 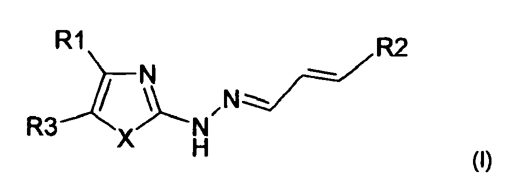 dans laquelle
X représente un hétéroatome choisi parmi l'oxygène, le soufre ou l'azote, l'atome d'azote étant éventuellement substitué par un groupement un radical alkyle ayant de 1 à 6 atomes de carbone, linéaire ou ramifié, éventuellement substitué par un atome d'halogène, un radical hydroxyle, un groupement amino, un groupement acide carboxylique;
R1 représente un groupement
- un radical alkyle ayant de 1 à 6 atomes de carbone, linéaire ou ramifié, éventuellement substitué par un atome d'halogène, un radical hydroxyle, un groupement amino, un groupement acide carboxylique,
- un radical dérivé d'un noyau carboné saturé cyclique ayant de 3 à 6 atomes de carbone, éventuellement substitué par un ou plusieurs atomes d'halogène, par un ou plusieurs radicaux alkyles ayant de 1 à 4 atomes de carbone, linéaire ou ramifié,
- un radical dérivé d'un noyau carboné aromatique cyclique ou polycyclique ayant de 6 à 10 atomes de carbone, éventuellement substitué une ou plusieurs fois par un ou plusieurs substituants choisis parmi un atome d'halogène, un radical alkyle ayant de 1 à 4 atomes de carbone, linéaire ou ramifié, éventuellement substitué par un atome ou plusieurs atomes d'halogène, par un radical hydroxyle, par un groupement amino, ou par un radical alkoxy ayant de 1 à 3 atomes de carbone, un radical hydroxyle, un groupement nitro, un groupement cyano, un groupement 1,3-dioxolyle, un groupement carbonyle, un groupement méthylsulfone ou un groupement amino éventuellement mono ou disubstitué par une chaîne alkyle de 1 à 3 atomes de carbone ou
- un radical mono cyclique aromatique dont le cycle présente 5 ou 6 atomes et comportant soit 1 ou 2 atomes d'azote soit 1 atome d'oxygène.;
R2 représente un groupement
- un radical alkyle ayant de 1 à 6 atomes de carbone, linéaire ou ramifié, éventuellement substitué par un atome d'halogène, un radical hydroxyle, un groupement amino, un groupement acide carboxylique,
- un radical dérivé d'un noyau carboné saturé cyclique ayant de 3 à 6 atomes de carbone, éventuellement substitué par un ou plusieurs atomes d'halogène, par un ou plusieurs radicaux alkyles ayant de 1 à 4 atomes de carbone, linéaire ou ramifié ou
- un radical dérivé d'un noyau carboné aromatique cyclique ou polycyclique ayant de 6 à 10 atomes de carbone, éventuellement substitué une ou plusieurs fois par un ou plusieurs substituants choisis parmi un atome d'halogène, un radical alkyle ayant de 1 à 4 atomes de carbone, linéaire ou ramifié, éventuellement substitué par un atome ou plusieurs atomes d'halogène, par un radical hydroxyle, par un groupement amino, ou par un radical alkoxy ayant de 1 à 3 atomes de carbone, un radical hydroxyle, un groupement nitro, un groupement cyano, un groupement 1,3-dioxolyle, un groupement carbonyle, un groupement méthylsulfone ou un groupement amino éventuellement mono ou disubstitué par une chaîne alkyle de 1 à 3 atomes de carbone ou
- un radical mono cyclique aromatique dont le cycle présente 5 ou 6 atomes et comporte 1 ou 2 atomes d'azote.
R3 représente un atome d'hydrogène ou un groupement
- un radical alkyle ayant de 1 à 6 atomes de carbone, linéaire ou ramifié, éventuellement substitué par un atome d'halogène, un radical hydroxyle, un groupement amino, un groupement acide carboxylique ou
- un radical dérivé d'un noyau carboné aromatique cyclique ou polycyclique ayant de 6 à 10 atomes de carbone, éventuellement substitué une ou plusieurs fois par un ou plusieurs substituants choisis parmi un atome d'halogène, un radical alkyle ayant de 1 à 4 atomes de carbone, linéaire ou ramifié, éventuellement substitué par un atome ou plusieurs atomes d'halogène, par un radical hydroxyle, par un groupement amino, ou par un radical alkoxy ayant de 1 à 3 atomes de carbone, un radical hydroxyle, un groupement nitro, un groupement cyano, un groupement 1,3-dioxolyle, un groupement carbonyle, un groupement méthylsulfone ou un groupement amino éventuellement mono ou disubstitué par une chaîne alkyle de 1 à 3 atomes de carbone
à l'exception des composés
- 3-phényl-2-propénal-[4-(4-nitrophényt)-2-thiazolyl] hydrazone;
- 3-phènyl-2-pmpènal-[4-(4-méthoxyphényl)-2-thiazolyl] hydrazone;
- 3-phényl-2-propénal-[4-(4-fluorophényl)-2-thiazolyl] hydrazone;
- 3-phényl-2-propénal-[4-(4-bromophényl)-2-thiazolyl] hydrazone;
- 3-phényl-2-propénal-[4-(4-chlorophényl)-2-thiazolyl] hydrazone;
- 3-phényl-2-propénal-[4-(4-méthylphényl)-2-thiazolyl] hydrazone;
- 3-phényl-2-propénal-[4-(1-naphthalènyl)-2-thiazolyl] hydrazone;
- 3-phényl-2-propénal-(5-méthyl-4-phényl-2-thiazolyl) hydrazone;
- 3-phényl-2-propénal-[4-(2,4-dichlorophényl)-2-thiazolyl] hydrazone;
- 3-phényl-2-propénal-[4-(3-nitrophényl)-2-thiazolyl] hydrazone;
- 3-phényl-2-propénal-[4-(1,1-diméthyléthyl)-2-thiazolyl] hydrazone;
- 3-phényl-2-propénal-[4-(4-chlorophényl)-5-phényl-2-thiazolyl] hydrazone;
- 2-buténal-[4-(4-nitrophényl)-2-thiazolyl] hydrazone;
- 3-phényl-2-propénal-[5-(4-chlorophényl)-4-phényl-2-thiazolyl] hydrazone;
- 3-(4-nitrophényl)-2-propénal-(4,5-diphényl-2-thiazolyl) hydrazone;
- 3-phényl-2-propénal-(4,5-diphényl-2-thiazolyl) hydrazone;
- acide 2-[[3-(3-nitrophényl)-2-propénylidène]hydrazino]-4-thiazole carboxylique;
- acide 2-[[3-(2-nitrophényl)-2-propénylidène]hydrazino]-4-thiazole carboxylique;
- 3-phényl-2-propénal-[4-chlorométhyl)-2-thiazolyl] hydrazone;
- cinnamaldéhyde-(4-phényl-2-thiazolyl) hydrazone;
- crotonaldéhyde-(4-phényl-2-thiazolyl) hydrazone;
- 2-furanacroléine-(4-méthyl-2-thiazolyl) hydrazone;
- cinnamaldéhyde-(4-méthyl-2-thiazolyl) hydrazone;
ainsi que leurs sels d'addition avec les acides pharmaceutiquement acceptables.

2. Composé de formule I selon la revendication 1, **caractérisé en ce que** X est un atome de soufre.

3. Composé de formule I selon l'une quelconque des revendications 1 ou 2, **caractérisé en ce que** R1 est un radical phényle substitué en position para par un atome de chlore, par un groupement méthyle ou trifluorométhyle ou en position méta par un groupement hydroxyle ou un radical phényle disubstitué en ortho par un groupement méthoxy et en para par un atome de chlore ou en ortho par un groupement méthyle et en para par un groupement hydroxyle ou en méta par un atome de fluor et en para par un atome de chlore.

4. Composé de formule I selon l'une quelconque des revendications 1 à 3, **caractérisé en ce que** R2 est un radical pyridin-4-yle, pyridin-2-yle ou pyridin-3-yle ou un radical benzo[1,3]dioxol-5-yle ou un radical phényle, ou phényle substitué par un groupement nitro, cyano, méthoxy, amino, méthylsulfone, amide, méthylcétone, hydroxyméthyle ou hydroxyle en position méta, ou phényle substitué en position ortho par un groupement méthoxy, méthyle, un atome de fluor ou de chlore, ou phényle substitué en position para par un atome de fluor.

5. Composé de formule I selon l'une quelconque des revendications 1 à 4, **caractérisé en ce que** R3 est un atome d'hydrogène ou un groupement méthyle.

6. Composé de formule I selon l'une quelconque des revendications 1 à 5, **caractérisé en ce que**
- X représente un atome de soufre,
- R3 est un atome d'hydrogène,
- R1 est un radical phényle substitué en position para par un atome de chlore, par un groupement trifluorométhyle, ou en position méta par un groupement hydroxyle, ou un radical phényle disubstitué en ortho par un groupement méthoxy et en para par un atome de chlore, ou en ortho par un groupement méthyle et en para par un groupement hydroxyle, ou en méta par un atome de fluor et en para par un atome de chlore,
- R2 est un radical pyridin-4-yle, pyridin-2-yle ou pyridin-3-yle, ou un radical benzo[1,3]dioxol-5-yle, ou un radical phényle, ou phényle substitué par un groupement nitro, cyano, amino, méthylsulfone, amide, méthylcétone, hydroxyméthyle hydroxyle ou méthoxy en position méta, ou phényle substitué en position ortho par un groupement méthoxy, méthyle, un atome de fluor ou de chlore, ou phényle substitué en position para par un atome de fluor.

7. Composé de formulé I selon l'une quelconque des revendications 1 à 6, **caractérisé en ce qu'**il s'agit de
- la 3-(2-fluorophényl)-2-propénal-[4-(4-chlorophényl-2-thiazolyl) hydrazone;
- la 3-(3-nitrophényl)-2-propénal-[4-(4-chlorophényl-2-thiazolyl) hydrazone;
- la 3-(2-méthoxyphényl)-2-propénal-[4-(4-chlorophényl-2-thiazolyl) hydrazone;
- la 3-(3-méthoxyphényl)-2-propénal-[4-(4-chlorophényl-2-thiazolyl) hydrazone;
- la 3-phényl-2-propénal-[4-(3-hydroxyphényl-2-thiazolyl) hydrazone;
- la 3-pyridin-4-yle-2-propénal-[4-(4-chlorophényl-2-thiazolyl) hydrazone;
- la 3-(2-méthylphényl)-2-propénal-[4-(4-chlorophényl-2-thiazolyl) hydrazone;
- la 3-(3-cyanophényl)-2-propénal-[4-(4-chlorophényl-2-thiazolyl) hydrazone;
- la 3-(2-chlorophényl)-2-propénal-[4-(4-chlorophényl-2-thiazolyl) hydrazone;
- la 3-(3-aminophényl)-2-propénal-[4-(4-chlorophényl-2-thiazolyl) hydrazone;
- la 3-(3-nitrophényl)-2-propénal-[4-(4-trifluorométhylphényl-2-thiazolyl) hydrazone;
- le 3-((E)-3-{[4-(4-chlorophényl)-thiazol-2-yl]-hydrazono}-propényl)-phénol;
- la 3-pyridin-4-yl-2-propénal-[4-(4-trifluorométhylphényl-2-thiazolyl) hydrazone;
- la 3-(3-aminophényl)-2-propénal-[4-(4-trifluorométhylphényl-2-thiazolyl) hydrazone;
- la 3-(3-méthanesulfonylphényl)-2-propénal-[4-(4-chlorophényl-2-thiazolyl) hydrazone;
- la 3-(3-cyanophényl)-2-propénal-[4-(4-hydroxyphényl-2-thiazolyl) hydrazone;
- la 3-(3-benzo[1,3]dioxol-5-yl)-2-propénal-[4-(4-trifluorométhylphényl-2-thiazolyl) hydrazone;
- la 3-phényl-2-propénal-[4-[4-chloro-2-méthoxyphényl-2-thiazolyl) hydrazone;
- la 1-[3-((E)-3-{[4-(4-trifluorométhylphényl)-thiazol-2-yl]-hydrazono}-propényl)-phényl]-éthanone;
- la 3-pyridin-2-yl-2-propénal-[4-(4-chlorophényl-2-thiazolyl) hydrazone;
- la 3-pyridin-2-yl-2-propénal-[4-(4-trifluorométhylphényl-2-thiazolyl) hydrazone;
- le 3-méthyl-4-(2-(N'-[(E)-3-phénylprop-2-èn-(E)-ylidène]hydrazino}-thiazol-4-yl)-phénol;
- le [3-((E)-3-{[4-(4-trifluorométhylphényl)-thiazol-2-yl]-hydrazono}-propènyl)-phényl]-méthanol;
- la 3-(4-fluorophényl)-2-propénal-[4-(4-trifluorométhylphényl-2-thiazolyl) hydrazone;
- la 3-((E)-{[4-(4-trifluorométhylphényl)-thiazol-2-yl]-hydrazono}-propènyl)-benzamide;
et tout particulièrement la 3-phényl-2-propénal-[4-(4-trifluorométhylphényl)-2-thiazolyl]hydrazone; le 3-((E)-3-{[4-(4-triflurométhylphényl)-thiazol-2-yl]-hydrazono}-propényl)-phénol; la 3-(3-méthanesulfonylphényl)-2-propénal-[4-(4-trifluorométhylphényl-2-thiazolyl)hydrazone; et la 3-(3-cyanophényl)-2-propénal-[4-(4-trifluorométhylphényl-2-thiazolyl)hydrazone.

8. Procédé de préparation d'un composé de formule I tel que décrit dans la revendication 1 et dans lequel X représente un atome de soufre, R1, R2 et R3 ayant les significations précédemment décrites aux revendications 1 à 7, dans lequel on fait réagir une ⊐-bromo-cétone de formule II 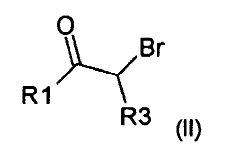 dans laquelle R1 et R3 peuvent avoir les significations précédemment indiquées avec un composé de formule III, 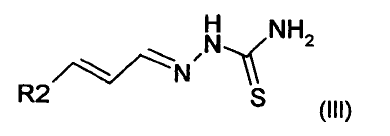 dans laquelle R2 peut avoir la signification précédemment indiquée dans un minimum de solvant adapté, pour obtenir le composé de formule I attendu.

9. Composition, notamment composition pharmaceutique ou médicament, comprenant au moins un composé de formule I, tels que décrits dans l'une quelconque des revendications 1 à 7, à l'exception des composés
- 3-phényl-2-propénal-[4-(4-nitrophényl)-2-thiazolyl] hydrazone;
- 3-phényl-2-propénal-[4-(4-méthoxyphényl)-2-thiazolyl] hydrazone;
- 3-phényl-2-propénal-[4-(4-fluorophényl)-2-thiazolyl] hydrazone;
- 3-phényl-2-propénal-[4-(4-bromophényl)-2-thiazolyl] hydrazone;
- 3-phényl-2-propénal-[4-(4-chlorophényl)-2-thiazolyl] hydrazone;
- 3-phényl-2-propénal-[4-(4-méthylphényl)-2-thiazolyl] hydrazone;
- 3-phényl-2-propénal- [4-(1-naphthalènyl)-2-thiazolyl] hydrazone;
- 3-phényl-2-propénal-(5-méthyl-4-phényl-2-thiazolyl) hydrazone;
- 3-phényl-2-propénal-[4-(2,4-dichlorophényl)-2-thiazolyl] hydrazone;
- 3-phényl-2-propénal-[4-(3-nitrophényl)-2-thiazolyl] hydrazone;
- 3-phényl-2-propénal-[4-(1,1-diméthyléthyl)-2-thiazolyl] hydrazone;
- 3-phényl-2-propénal-[4-(4-chlorophényl)-5-phényl-2-thiazolyl] hydrazone;
- 2-buténal-[4-(4-nitrophényl)-2-thiazolyl] hydrazone;
- 3-phényl-2-propénal-[5-(4-chlorophényl)-4-phényl-2-thiazolyl] hydrazone;
- 3-(4-nitrophényl)-2-propénal-(4,5-diphényl-2-thiazolyl) hydrazone;
- 3-phényl-2-propénal-(4,5-diphényl-2-thiazolyl) hydrazone;
- acide 2-[[3-(3-nitrophényl)-2-propénylidène]hydrazino]-4-thiazole carboxylique;
- acide 2-[[3-(2-nitrophényl)-2-propénylidènejhydrazino]-4-thiazole carboxylique;
- 3-phényl-2-propénal-[4-chlorométhyl)-2-thiazolyl] hydrazone;
- cinnamaldéhyde-(4-phényl-2-thiazolyl) hydrazone;
- crotonaldéhyde-(4-phényl-2-thiazolyl) hydrazone;
- 2-furanacroléine-(4-méthyl-2-thiazolyl) hydrazone;
- cinnamaldéhyde-(4-méthyl-2-thiazolyl) hydrazone;
ainsi que leurs sels d'addition avec les acides pharmaceutiquement acceptables.

10. Utilisation des composés de formule I 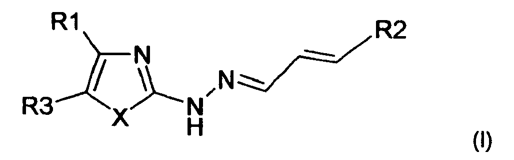 dans laquelle X, R1, R2 et R3 peuvent avoir les significations précédemment décrites dans les revendications 1 à 7 y compris la
- 3-phényl-2-propénal-[4-(4-nitrophényl)-2-thiazolyl] hydrazone;
- 3-phényl-2-propénal-[4-(4-méthoxyphényl)-2-thiazolyl] hydrazone;
- 3-phényl-2-propénal-[4-(4-fluorophényl)-2-thiazolyl] hydrazone;
- 3-phényl-2-propénal-[4-(4-bromophényl)-2-thiazolyl] hydrazone;
- 3-phényl-2-propénal-[4-(4-chlorophényl)-2-thiazolyl] hydrazone;
- 3-phényl-2-propénal-[4-(4-méthylphényl)-2-thiazolyl] hydrazone;
- 3-phényl-2-propénal- [4-(1-naphthalènyl)-2-thiazolyl] hydrazone;
- 3-phényl-2-propénal-(5-méthyl-4-phényl-2-thiazolyl) hydrazone;
- 3-phényl-2-propénal-[4-(2,4-dichlorophényl)-2-thiazolyl] hydrazone;
- 3-phényl-2-propénal-[4-(3-nitrophényl)-2-thiazolyl] hydrazone;
- 3-phényl-2-propénal-[4-(1,1-diméthyléthyl)-2-thiazolyl] hydrazone;
- 3-phényl-2-propénal-[4-(4-chlorophényl)-5-phényl-2-thiazolyl] hydrazone;
- 2-buténal-[4-(4-nitrophényl)-2-thiazolyl] hydrazone;
- 3-phényl-2-propénal-[5-(4-chlorophényl)-4-phényl-2-thiazolyl] hydrazone;
- 3-(4-nitrophényl)-2-propénal-(4,5-diphényl-2-thiazolyl) hydrazone;
- 3-phényl-2-propénal-(4,5-diphényl-2-thiazolyl) hydrazone;
- acide 2-[[3-(3-nitrophényl)-2-propénylidène]hydrazino]-4-thiazole carboxylique;
- acide 2-[[3-(2-nitrophényl)-2-propénylidène]hydrazino]-4-thiazole carboxylique;
- 3-phényl-2-propénal-[4-chlorométhyl)-2-thiazolyl] hydrazone;
- cinnamaldéhyde-(4-phényl-2-thiazolyl) hydrazone;
- crotonaldéhyde-(4-phényl-2-thiazolyl) hydrazone;
- 2-furanacroléine-(4-méthyl-2-thiazolyl) hydrazone;
dans la préparation d'un médicament.

11. Utilisation selon la revendication 10, **caractérisée en ce que** le composé de formule I est choisi parmi
- la 3-(2-fluorophényl)-2-propénal-[4-(4-chlorophényl-2-thiazolyl) hydrazone;
- la 3-(3-nitrophényl)-2-propénal-[4-(4-chlorophényl-2-thiazolyl) hydrazone;
- la 3-(2-méthoxyphényl)-2-propénal-[4-(4-chlorophényl-2-thiazolyl) hydrazone;
- la 3-(3-méthoxyphényl)-2-propénal-[4-(4-chlorophényl-2-thiazolyl) hydrazone;
- la 3-phényl-2-propénal-[4-(3-hydroxyphényl-2-thiazolyl) hydrazone;
- la 3-pyùdin-4-yle-2-pmpénal-[4-(4-chlorophényph-thiazolyl) hydrazone;
- la 3-(2-méthylphényl)-2-propénal-[4-(4-chlorophényl-2-thiazolyl) hydrazone;
- la 3-(3-cyanophényl)-2-propénal-[4-(4-chlorophényl-2-thiazolyl) hydrazone;
- la 3-(2-chlorophényl)-2-propénal-[4-(4-chlorophényl-2-thiazolyl) hydrazone;
- la 3-(3-aminophény)-2-propénal-[4-(4-chlorophényl-2-thiazolyl) hydrazone;
- la 3-(3-nitrophényl)-2-propénal-[4-(4-trifluomméthylphényl-2-thiazolyl) hydrazone;
- le 3-((E)-3-{[4-(4-chlorophényl)-thiazol-2-yl]-hydrazono}propényl)-phénol;
- la 3-pyridin-4-yl-2-propénal-[4-(4-trifluorométhylphényl-2-thiazolyl) hydrazone;
- la 3-(3-aminophényl)-2-propénal-[4-(4-trifluorométhylphényl-2-thiazolyl) hydrazone;
- la 3-(3-méthanesulfonylphényl)-2-propénal-[4-(4-chlorophényl-2-thiazolyl) hydrazone;
- la 3-(3-cyanophényl)-2-propénal-[4-(4-hydroxyphényl-2-thiazolyl) hydrazone;
- la 3-(3-benzo[1,3]dioxol-5-yl)-2-propénal-[4-(4-trifluorométhylphényl-2-thiazolyl) hydrazone;
- la 3-phényl-2-propénal-[4-(4-chloro-2-méthoxyphényl-2-thiazolyl) hydrazone;
- la 1-[3-((E)-3-{[4-(4-trifluorométhylphényl)-thiazol-2-yl]-hydrazono}-propényl)-phényl]-éthanone;
- la 3-pyridin-2-yl-2-propénal-[4-(4-chlorophényl-2-thiazolyl) hydrazone;
- la 3-pyridin-2-yl-2-propénal-[4-(4-trifluorométhylphényl-2-thiazolyl) hydrazone;
- le 3-méthyl-4-(2-{N'-[(E)-3-phénylprop-2-èn-(E)-ylidène)hydrazino}thiazol-4-yl)-phénol;
- le [3-((E)-3-{[4-(4-trifluorométhylphényl)-thiazol-2-yl]-hydmzono}-propènyl)-phényl]-méthanol;
- la 3-(4-fluorophényl)-2-propénal-[4-(4-trifluorométhylphényl-2-thiazolyl) hydrazone;
- la 3-((E)-3-{[4-(4-trifluorométhylphényl)-thiazol-2-yl]-hydrazono}-propènyl)-benzamide;
et tout particulièrement la 3-phényl-2-propénal-[4-(4-trifluorométhylphényl)-2-thiazolyl]hydrazone; le 3-((E)-3-{[4-(4-trifluorométhylphényl)-thiazol-2-yl]-hydrazono}-propényl)-phénol; la 3-(3-méthanesulfonylphényl)-2-propénal-[4-(4-trifluorométhylphényl-2-thiazolyl)hydrazone; et la 3-(3-cyanophényl)-2-propénal-[4-(4-trifluorométhylphényl-2-thiazolyl)hydrazone.

12. Utilisation des composés de formule I tels que décrits dans l'une quelconque des revendications 10 ou 11, ou de leurs sels d'addition avec les acides pharmaceutiquement acceptables, y compris la
- 3-phényl-2-propénal-[4-[4-nitrophényl)-2-thiazolyl] hydrazone;
- 3-phényl-2-propénal-[4-(4-méthoxyphényl)-2-thiazolyl] hydrazone;
- 3-phényl-2-propénal-[4-(4-fluorophényl)-2-thiazolyl] hydrazone;
- 3-phényl-2-propénal-[4-(4-bromophényl)-2-thiazolyl] hydrazone;
- 3-phényl-2-propénal-[4-(4-chlorophényl)-2-thiazolyl] hydrazone;
- 3-phényl-2-propénal-[4-(4-méthylphényl)-2-thiazolyl] hydrazone;
- 3-phényl-2-propénal- [4-(1-naphthalènyl)-2-thiazolyl] hydrazone;
- 3-phényl-2-propénal-(5-méthyl-4-phényl-2-thiazolyl) hydrazone;
- 3-phényl-2-propénal-[4-(2,4-dichlorophényl)-2-thiazolyl] hydrazone;
- 3-phényh2-propénal-[4-(3-nitrophényl)-2-thiazolyl] hydrazone;
- 3-phényl-2-propénal-[4-(1,1-diméthyléthyl)-2-thiazolyl] hydrazone;
- 3-phényl-2-propénal-[4-(4-chlorophényl)-5-phényl-2-thiazolyl] hydrazone;
- 2-buténal-[4-(4-nitrophényl)-2-thiazolyl] hydrazone;
- 3-phényl-2-propénal-[5-(4-chlorophényl)-4-phényl-2-thiazolyl] hydrazone;
- 3-(4-nitrophényl)-2-propénal-(4,5-diphényl-2-thiazolyl) hydrazone;
- 3-phényl-2-propénal-(4,5-diphényl-2-thiazolyl) hydrazone;
- acide 2-[[3-(3-nitrophényl)-2-propénylidène]hydrazino]-4-thiazole carboxylique;
- acide 2-[[3-(2-nitrophényl)-2-propénylidène]hydrazino]-4-thiazole carboxylique;
- 3-phényl-2-propénal-[4-chlorométhyl)-2-thiazolyl] hydrazone;
- cinnamaldéhyde-(4-phényl-2-thiazolyl) hydrazone;
- crotonaldéhyde-(4-phényl-2-thiazolyl) hydrazone;
- 2-furanacroléine-(4-méthyl-2-thiazolyl) hydrazone;
- cinnamaldéhyde-(4-méthyl-2-thiazolyl) hydrazone;
dans la préparation d'un médicament destiné au traitement des maladies chroniques neurodégénératives.

13. Utilisation selon la revendication 12, **caractérisée en ce que** le médicament est destiné au traitement des pathologies neurodégénératives à polyglutamines.

14. Utilisation selon l'une quelconque des revendications 12 ou 13, **caractérisée en ce que** le médicament est destiné au traitement de la maladie de Huntington.

15. Utilisation des composés de formule I, tels que décrits dans l'une quelconque des revendications 10 ou 11, ou de leurs sels d'addition avec les acides pharmaceutiquement acceptables, y compris la
- 3-phényl-2-propénal-[4-(4-nitrophényl)-2-thiazolyl] hydrazone;
- 3-phényl-2-propénal-[4-(4-méthoxyphényl)-2-thiazolyl] hydrazone;
- 3-phényl-2-propénal-[4-(4-fluorophényl)-2-thiazolyl] hydrazone;
- 3-phényl-2-propénal-[4-(4-bromophényl)-2-thiazolyl] hydrazone;
- 3-phényl-2-propénal-[4-(4-chlorophényl)-2-thiazolyl] hydrazone;
- 3-phényl-2-propénal-[4-(4-méthylphényl)-2-thiazolyl] hydrazone;
- 3-phényl-2-propénal- [4-(1-naphthalènyl)-2-thiazolyl] hydrazone;
- 3-phényl-2-propénal-(5-méthyl-4-phényl-2-thiazolyl) hydrazone;
- 3-phényl-2-propénal-[4-(2,4-dichlorophényl)-2-thiazolyl] hydrazone;
- 3-phényl-2-propénal-[4-(3-nitrophényl)-2-thiazolyl] hydrazone;
- 3-phényl-2-propénal-[4-(1,1-diméthyléthyl)-2-thiazolyl] hydrazone;
- 3-phènyl-2-propènal-[4-(4-chlorophènyl)-5-phènyl-2-thiazolyl] hydrazone;
- 2-buténal-[4-(4-nitrophényl)-2-thiazolyl] hydrazone;
- 3-phényl-2-propénal-[5-(4-chlorophényl)-4-phényl-2-thiazolyl] hydrazone;
- 3-(4-nitrophényl)-2-propénal-(4,5-diphényl-2-thiazolyl) hydrazone;
- 3-phényl-2-propénal-(4,5-diphényl-2-thiazolyl) hydrazone;
- acide 2-[[3-(3-nitrophényl)-2-propénylidène]hydrazino]-4-thiazole carboxylique
- acide 2-[[3-(2-nitrophényl)-2-propénylidène]hydrazino]-4-thiazole carboxylique
- 3-phényl-2-propénal-[4-chlorométhyl)-2-thiazolyl] hydrazone;
- cinnamaldéhyde-(4-phényl-2-thiazolyl) hydrazone;
- crotonaldéhyde-(4-phényl-2-thiazolyl) hydrazone;
- 2-furanacroléine-(4-méthyl-2-thiazolyl) hydrazone;
- cinnamaldéhyde-(4-méthyl-2-thiazolyl) hydrazone;
dans la préparation d'un médicament destinée au traitement des maladies chroniques neurodégénératives, héréditaires ou sporadiques, notamment la maladie d'Alzheimer (AD), la maladie de Parkinson (PD), l'atrophie musculaire spino-bulbaire ou KENNEDY, les démences à corps de Lewy, les ataxies spino-cérébelleuses, la sclérose latérale amyotrophique (ALS), les amyotrophies spinales (SMA), la maladie de Creutzfeldt-Jakob, la sclérose en plaque (MS), l'adrénoleucodystrophie, l'épilepsie, les démences, la schizophrénie, la DRPLA, les syndromes neurologiques associés au syndrome d'immuno-déficience acquis (SIDA), les lésions neuronales liées au vieillissement, les neuropathies périphériques héréditaires ou lésionnelles, comme les maladies de Fabry, de Charcot-Marie-Tooth, de Krabbe, les leucodystrophies, les neuropathies diabétiques et celles induites par les traitements anti-cancéreux, les traumatismes du cerveau, des nerfs périphériques ou de la moëlle épinière, les ischémies du cerveau ou de la moëlle épinière suite à un accident cérébro-vasculaire, ou induites par un manque d'irrigation sanguine, les dégénérescences, héréditaires, lésionnelles ou liées au vieillissement des neurones sensoriels de la vision, comme les dégénérescences maculaires, les rétinites pigmentaires, ou les dégénérescences du nerf optique induites par les glaucomes, les dégénérescences, héréditaires, traumatiques ou liées au vieillissement des neurones sensoriels de l'ouïe entraînant une diminution ou une perte de l'audition.

## Claims

1. Novel chemical compounds corresponding to formula I 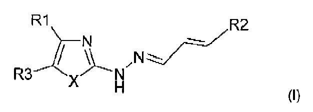 in which
X represents a heteroatom chosen from oxygen, sulphur or nitrogen, the nitrogen atom being optionally substituted by a group:
- a linear or branched alkyl radical having 1 to 6 carbon atoms, optionally substituted by a halogen atom, a hydroxyl radical, an amino group, a carboxylic acid group;
R1 represents a group:
- a linear or branched alkyl radical having 1 to 6 carbon atoms, optionally substituted by a halogen atom, a hydroxyl radical, an amino group, a carboxylic acid group,
- a radical derived from a cyclic saturated carbon-containing ring having 3 to 6 carbon atoms, optionally substituted by one or more halogen atoms, by one or more linear or branched alkyl radicals having 1 to 4 carbon atoms,
- a radical derived from a cyclic or polycyclic aromatic carbon-containing ring having 6 to 10 carbon atoms, optionally substituted one or more times by one or more substituents chosen from a halogen atom, a linear or branched alkyl radical having 1 to 4 carbon atoms, optionally substituted by one or more halogen atoms, by a hydroxyl radical, by an amino group, or by an alkoxy radical having 1 to 3 carbon atoms, a hydroxyl radical, a nitro group, a cyano group, a 1,3-dioxolyl group, a carbonyl group, a methyl sulphone group or an amino group optionally mono- or disubstituted by an alkyl chain of 1 to 3 carbon atoms or
- an aromatic monocyclic radical the ring of which has 5 or 6 atoms and comprising either 1 or 2 nitrogen atoms or 1 oxygen atom;
R2 represents a group:
- a linear or branched alkyl radical having 1 to 6 carbon atoms, optionally substituted by a halogen atom, a hydroxyl radical, an amino group, a carboxylic acid group,
- a radical derived from a cyclic saturated carbon-containing ring having 3 to 6 carbon atoms, optionally substituted by one or more halogen atoms, by one or more linear or branched alkyl radicals having 1 to 4 carbon atoms, or
- a radical derived from a cyclic or polycyclic aromatic carbon-containing ring having 6 to 10 carbon atoms, optionally substituted one or more times by one or more substituents chosen from a halogen atom, a linear or branched alkyl radical having 1 to 4 carbon atoms, optionally substituted by one or more halogen atoms, by a hydroxyl radical, by an amino group, or by an alkoxy radical having 1 to 3 carbon atoms, a hydroxyl radical, a nitro group, a cyano group, a 1,3-dioxolyl group, a carbonyl group, a methyl sulphone group or an amino group optionally mono- or disubstituted by an alkyl chain of 1 to 3 carbon atoms or
- an aromatic monocyclic radical the ring of which has 5 or 6 atoms and comprises 1 or 2 nitrogen atoms.
R3 represents a hydrogen atom or a group:
- a linear or branched alkyl radical having 1 to 6 carbon atoms, optionally substituted by a halogen atom, a hydroxyl radical, an amino group, a carboxylic acid group or
- a radical derived from a cyclic or polycyclic aromatic carbon-containing ring having 6 to 10 carbon atoms, optionally substituted one or more times by one or more substituents chosen from a halogen atom, a linear or branched alkyl radical having 1 to 4 carbon atoms, optionally substituted by one or more halogen atoms, by a hydroxyl radical, by an amino group, or by an alkoxy radical having 1 to 3 carbon atoms, a hydroxyl radical, a nitro group, a cyano group, a 1,3-dioxolyl group, a carbonyl group, a methyl sulphone group or an amino group optionally mono- or disubstituted by an alkyl chain of 1 to 3 carbon atoms
with the exception of the compounds
- 3-phenyl-2-propenal-[4-(4-nitrophenyl)-2-thiazolyl] hydrazone;
- 3-phenyl-2-propenal-[4-(4-methoxyphenyl)-2-thiazolyl] hydrazone;
- 3-phenyl-2-propenal-[4-(4-fluorophenyl)-2-thiazolyl] hydrazone;
- 3-phenyl-2-propenal-[4-(4-bromophenyl)-2-thiazolyl] hydrazone;
- 3-phenyl-2-propenal-[4-(4-chlorophenyl)-2-thiazolyl] hydrazone;
- 3-phenyl-2-propenal-[4-(4-methylphenyl)-2-thiazolyl] hydrazone;
- 3-phenyl-2-propenal- [4-(1-naphthalenyl)-2-thiazolyl] hydrazone;
- 3-phenyl-2-propenal-(5-methyl-4-phenyl-2-thiazolyl) hydrazone;
- 3-phenyl-2-propenal-[4-(2,4-dichlorophenyl)-2-thiazolyl] hydrazone;
- 3-phenyl-2-propenal-[4-(3-nitrophenyl)-2-thiazolyl] hydrazone;
- 3-phenyl-2-propenal-[4-(1,1-dimethylethyl)-2-thiazolyl] hydrazone;
- 3-phenyl-2-propenal-[4-(4-chlorophenyl)-5-phenyl-2-thiazolyl] hydrazone;
- 2-butenal-[4-(4-nitrophenyl)-2-thiazolyl] hydrazone;
- 3-phenyl-2-propenal-[5-(4-chlorophenyl)-4-phenyl-2-thiazolyl] hydrazone;
- 3-(4-nitrophenyl)-2-propenal-(4,5-diphenyl-2-thiazolyl) hydrazone;
- 3-phenyl-2-propenal-(4,5-diphenyl-2-thiazolyl) hydrazone;
- 2-[[3-(3-nitrophenyl)-2-propenylidene]hydrazino]-4-thiazole carboxylic acid;
- 2-[[3-(2-nitrophenyl)-2-propenylidene]hydrazino]-4-thiazole carboxylic acid;
- 3-phenyl-2-propenal-[4-chloromethyl)-2-thiazolyl] hydrazone;
- cinnamaldehyde-(4-phenyl-2-thiazolyl) hydrazone;
- crotonaldehyde-(4-phenyl-2-thiazolyl) hydrazone;
- 2-furanacroleine-(4-methyl-2-thiazolyl) hydrazone;
- cinnamaldehyde-(4-methyl-2-thiazolyl) hydrazone;
as well as their addition salts with pharmaceutically acceptable acids.

2. Compound of formula I according to claim 7, **characterized in that** X is a sulphur atom.

3. Compound of formula I according to any one of claims 1 or 2, **characterized in that** R1 is a phenyl radical substituted in para position by a chlorine atom, by a methyl or trifluoromethyl group or in meta position by a hydroxyl group or a phenyl radical disubstituted in ortho position by a methoxy group and in para position by a chlorine atom or in ortho position by a methyl group and in para position by a hydroxyl group or in meta position by a fluorine atom and in para position by a chlorine atom

4. Compound of formula I according to any one of claims 1 to 3, **characterized in that** R2 is a pyridin-4-yl, pyridin-2-yl or pyridin-3-yl radical or a benzo[1,3]dioxol-5-yl radical or a phenyl radical, or phenyl substituted by a nitro, cyano, methoxy, amino, methyl sulphone, amide, methyl ketone, hydroxymethyl or hydroxyl group in meta position, or phenyl substituted in ortho position by a methoxy, methyl group, a fluorine or chlorine atom, or phenyl substituted in para position by a fluorine atom.

5. Compound of formula I according to any one of claims 1 to 4, **characterized in that** R3 is a hydrogen atom or a methyl group.

6. Compound of formula I according to any one of claims 1 to 5, **characterized in that**
- X represents a sulphur atom,
- R3 is a hydrogen atom,
- R1 is a phenyl radical substituted in para position by a chlorine atom, by a trifluoromethyl group, or in meta position by a hydroxyl group, or a phenyl radical disubstituted in ortho position by a methoxy group and in para position by a chlorine atom, or in ortho position by a methyl group and in para position by a hydroxyl group, or in meta position by a fluorine atom and in para position by a chlorine atom,
- R2 is a pyridin-4-yl, pyridin-2-yl or pyridin-3-yl radical, or a benzo[1,3]dioxol-5-yl radical, or a phenyl radical, or phenyl substituted by a nitro, amino, methyl sulphone, amide, methyl ketone, hydroxymethyl hydroxyl or methoxy cyano group in meta position, or phenyl substituted in ortho position by a methoxy, methyl group, a fluorine or chlorine atom, or phenyl substituted in para position by a fluorine atom.

7. Compound of formula I according to any one of claims 1 to 6, **characterized in that** it is
- 3-(2-fluorophenyl)-2-propenal-[4-(4-chlorophenyl-2-thiazolyl) hydrazone;
- 3-(3-nitrophenyl)-2-propenal-[4-(4-chlorophenyl-2-thiazolyl) hydrazone;
- 3-(2-methoxyphenyl)-2-propenal-[4-(4-chlorophenyl-2-thiazolyl) hydrazone;
- 3-(3-methoxyphenyl)-2-propenal-[4-(4-chlorophenyl-2-thiazolyl) hydrazone;
- 3-phenyl-2-propenal-[4-(3-hydroxyphenyl-2-thiazolyl) hydrazone;
- 3-pyridin-4-yl-2-propenal-[4-(4-chlorophenyl-2-thiazolyl) hydrazone;
- 3-(2-methylphenyl)-2-propenal-[4-(4-chlorophenyl-2-thiazolyl) hydrazone;
- 3-(3-cyanophenyl)-2-propenal-[4-(4-chlorophenyl-2-thiazolyl) hydrazone;
- 3-(2-chlorophenyl)-2-propenal-[4-(4-chlorophenyl-2-thiazolyl) hydrazone;
- 3-(3-aminophenyl)-2-propenal-[4-(4-chlorophenyl-2-thiazolyl) hydrazone;
- 3-(3-nitrophenyl)-2-propenal-[4-(4-trifluoromethylphenyl-2-thiazolyl) hydrazone;
- 3-((E)-3{[4-(4-chlorophenyl)-thiazol-2-yl]-hydrazono}-propenyl)-phenol;
- 3-pyridin-4-yl-2-propenal-[4-(4-trifluoromethylphenyl-2-thiazolyl) hydrazone;
- 3-(3-aminophenyl)-2-propenal-[4-(4-trifluoromethylphenyl-2-thiazolyl) hydrazone;
- 3-(3-methanesulphonylphenyl)-2-propenal-[4-(4-chlorophenyl-2-thiazolyl) hydrazone;
- 3-(3-cyanophenyl)-2-propenal-[4-(4-hydroxyphenyl-2-thiazolyl) hydrazone;
- 3-(3-benzo[1,3]dioxol-5-yl)-2-propenal-[4-(4-trifluoromethylphenyl-2-thiazolyl) hydrazone;
- 3-phenyl-2-propenal-[4-(4-chloro-2-methoxyphenyl-2-thiazolyl) hydrazone;
- 1-[3-((E)-3-{[4-(4-trifluoromethylphenyl)-thiazol-2-yl]-hydrazono}-propenyl)-phenyl]-ethanone;
- 3-pyridin-2-yl-2-propenal-[4-(4-chlorophenyl-2-thiazolyl) hydrazone;
- 3-pyridin-2-yl-2-propenal-[4-(4-trifluoromethylphenyl-2-thiazolyl) hydrazone;
- 3-methyl-4-(2-{N'-[(E)-3-phenylprop-2-en-(E)-ylidene]-hydrazino}-thiazol-4-yl)-phenol;
- [3-((E)-3-{[4-(4-trifluoromethylphenyl)-thiazol-2-yl]-hydrazono}-propenyl)-phenyl]-methanol;
- 3-(4-fluorophenyl)-2-propenal-[4-(4-trifluoromethylphenyl-2-thiazolyl) hydrazone;
- 3-((E)-3-{[4-(4-trifluoromethylphenyl)-thiazol-2-yl]-hydrazono}-propenyl)-benzamide;
and quite particularly 3-phenyl-2-propenal-[4-(4-trifluoromethylphenyl)-2-thiazolyl]hydrazone; 3-((E)-3-{[4-(4-trifluoromethylphenyl)-thiazol-2-yl]-hydrazono}-propenyl)-phenol; 3-(3-methanesulphonylphenyl)-2-propenal-[4-(4-trifluoromethylphenyl-2-thiazolyl)hydrazone; and 3-(3-cyanophenyl)-2-propenal-[4-(4-trifluoromethylphenyl-2-thiazolyl)hydrazone.

8. Process for the preparation of a compound of formula I as described in claim 1 and in which X represents a sulphur atom, R1, R2 and R3 having the meanings previously described in claims 1 to 7, in which a α-bromo-ketone of formula II 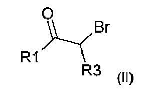 in which R1 and R3 can have the meanings previously indicated is reacted with a compound of formula III, 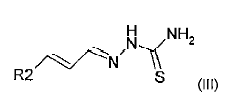 in which R2 can have the meaning previously indicated in a minimum amount of suitable solvent, in order to obtain the expected compound of formula I.

9. Composition, in particular pharmaceutical composition or medicament, comprising at least one compound of formula I, as described in any one of claims 1 to 7, with the exception of the compounds
- 3-phenyl-2-propenal-[4-(4-nitrophenyl)-2-thiazolyl] hydrazone;
- 3-phenyl-2-propenal-[4-(4-methoxyphenyl)-2-thiazolyl] hydrazone;
- 3-phenyl-2-propenal-[4-(4-fluorophenyl)-2-thiazolyl] hydrazone;
- 3-phenyl-2-propenal-[4-(4-bromophenyl)-2-thiazolyl] hydrazone;
- 3-phenyl-2-propenal-[4-(4-chlorophenyl)-2-thiazolyl] hydrazone;
- 3-phenyl-2-propenal-[4-(4-methylphenyl)-2-thiazolyl] hydrazone;
- 3-phenyl-2-propenal- [4-(1-naphthalenyl)-2-thiazolyl] hydrazone;
- 3-phenyl-2-propenal-(5-methyl-4-phenyl-2-thiazolyl) hydrazone:
- 3-phenyl-2-propenal-[4-(2,4-dichlorophenyl)-2-thiazolyl] hydrazone;
- 3-phenyl-2-propenal-[4-(3-nitrophenyl)-2-thiazolyl] hydrazone;
- 3-phenyl-2-propenal-[4-(1,1-dimethylethyl)-2-thiazolyl] hydrazone;
- 3-phenyl-2-propenal-[4-(4-chlorophenyl)-5-phenyl-2-thiazolyl] hydrazone;
- 2-butenal-[4-(4-nitrophenyl)-2-thiazolyl] hydrazone;
- 3-phenyl-2-propenal-[5-(4-chlorophenyl)-4-phenyl-2-thiazolyl] hydrazone;
- 3-(4-nitrophenyl)-2-propenal-(4,5-diphenyl-2-thiazolyl) hydrazone;
- 3-phenyl-2-propenal-(4,5-diphenyl-2-thiazolyl) hydrazone;
- 2-[[3-(3-nitrophenyl)-2-propenylidene]hydrazino]-4-thiazole carboxylic acid;
- 2-[[3-(2-nitrophenyl)-2-propenylidene]hydrazino]-4-thiazole carboxylic acid;
- 3-phenyl-2-propenal-[4-chloromethyl)-2-thiazolyl] hydrazone;
- cinnamaldehyde-(4-phenyl-2-thiazolyl) hydrazone;
- crotonaldehyde-(4-phenyl-2-thiazolyl) hydrazone;
- 2-furanacroleine-(4-methyl-2-thiazolyl) hydrazone;
- cinnamaldehyde-(4-methyl-2-thiazolyl) hydrazone;
as well as their addition salts with pharmaceutically acceptable acids.

10. Use of the compounds of formula I 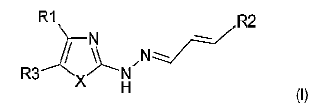 in which X, R1, R2 and R3 can have the meanings previously described in claims 1 to 7, including
- 3-phenyl-2-propenal-[4-(4-nitrophenyl)-2-thiazolyl] hydrazone;
- 3-phenyl-2-propenal-[4-(4-methoxyphenyl)-2-thiazolyl] hydrazone;
- 3-phenyl-2-propenal-[4-(4-fluorophenyl)-2-thiazolyl] hydrazone;
- 3-phenyl-2-propenal-[4-(4-bromophenyl)-2-thiazolyl] hydrazone;
- 3-phenyl-2-propenal-[4-(4-chlorophenyl)-2-thiazolyl] hydrazone;
- 3-phenyl-2-propenal-[4-(4-methylphenyl)-2-thiazolyl] hydrazone;
- 3-phenyl-2-propenal-[4-(1-naphthalenyl)-2-thiazolyl] hydrazone;
- 3-phenyl-2-propenal-(5-methyl-4-phenyl-2-thiazolyl) hydrazone;
- 3-phenyl-2-propenal-[4-(2,4-dichlorophenyl)-2-thiazolyl] hydrazone;
- 3-phenyl-2-propenal-[4-(3-nitrophenyl)-2-thiazolyl] hydrazone;
- 3-phenyl-2-propenal-[4-(1,1-dimethylethyl)-2-thiazolyl] hydrazone;
- 3-phenyl-2-propenal-[4-(4-chlorophenyl)-5-phenyl-2-thiazolyl] hydrazone;
- 2-butenal-[4-(4-nitrophenyl)-2-thiazolyl] hydrazone;
- 3-phenyl-2-propenal-[5-(4-chlorophenyl)-4-phenyl-2-thiazolyl] hydrazone;
- 3-(4-nitrophenyl)-2-propenal-(4,5-diphenyl-2-thiazolyl) hydrazone;
- 3-phenyl-2-propenal-(4,5-diphenyl-2-thiazolyl) hydrazone;
- 2-[[3-(3-nitrophenyl)-2-propenylidene]hydrazino]-4-thiazole carboxylic acid;
- 2-[[3-(2-nitrophenyl)-2-propenylidene]hydrazino]-4-thiazole carboxylic acid;
- 3-phenyl-2-propenal-[4-chloromethyl)-2-thiazolyl] hydrazone;
- cinnamaldehyde-(4-phenyl-2-thiazolyl) hydrazone;
- crotonaldehyde-(4-phenyl-2-thiazolyl) hydrazone;
- 2-furanacroleine-(4-methyl-2-thiazolyl) hydrazone;
in the preparation of a medicament.

11. Use according to claim 10, **characterized in that** the compound of formula I is chosen from
- 3-(2-fluorophenyl)-2-propenal-[4-(4-chlorophenyl-2-thiazolyl) hydrazone;
- 3-(3-nitrophenyl)-2-propenal-[4-(4-chlorophenyl-2-thiazolyl) hydrazone;
- 3-(2-methoxyphenyl)-2-propenal-[4-(4-chlorophenyl-2-thiazolyl) hydrazone;
- 3-(3-methoxyphenyl)-2-propenal-[4-(4-chlorophenyl-2-thiazolyl) hydrazone;
- 3-phenyl-2-propenal-[4-(3-hydroxyphenyl-2-thiazolyl) hydrazone;
- 3-pyridin-4-yl-2-propenal-[4-(4-chlorophenyl-2-thiazolyl) hydrazone;
- 3-(2-methylphenyl)-2-propenal-[4-(4-chlorophenyl-2-thiazolyl) hydrazone;
- 3-(3-cyanophenyl)-2-propenal-[4-(4-chlorophenyl-2-thiazolyl) hydrazone;
- 3-(2-chlorophenyl)-2-propenal-[4-(4-chlorophenyl-2-thiazolyl) hydrazone;
- 3-(3-aminophenyl)-2-propenal-[4-(4-chlorophenyl-2-thiazolyl) hydrazone;
- 3-(3-nitrophenyl)-2-propenal-[4-(4-trifluoromethylphenyl-2-thiazolyl) hydrazone;
- 3-((E)-3-{[4-(4-chlorophenyl)-thiazol-2-yl]-hydrazono}-propenyl)-phenol;
- 3-pyridin-4-yl-2-propenal-[4-(4-trifluoromethylphenyl-2-thiazolyl) hydrazone;
- 3-(3-aminophenyl)-2-propenal-[4-(4-trifluoromethylphenyl-2-thiazolyl) hydrazone;
- 3-(3-methanesulphonylphenyl)-2-propenal-[4-(4-chlorophenyl-2-thiazolyl) hydrazone;
- 3-(3-cyanophenyl)-2-propenal-[4-(4-hydroxyphenyl-2-thiazolyl) hydrazone;
- 3-(3-benzo[1,3]dioxol-5-yl)-2-propenal-[4-(4-trifluoromethylphenyl-2-thiazolyl) hydrazone;
- 3-phenyl-2-propenal-[4-(4-chloro-2-methoxyphenyl-2-thiazolyl) hydrazone;
- 1-[3-((E)-3-{[4-(4-trifluoromethylphenyl)-thiazol-2-yl]-hydrazono}-propenyl)-phenyl]-ethanone;
- 3-pyridin-2-yl-2-propenal-[4-(4-chlorophenyl-2-thiazolyl) hydrazone;
- 3-pyridin-2-yl-2-propenal-[4-(4-trifluoromethylphenyl-2-thiazolyl) hydrazone;
- 3-methyl-4-(2-{N'-[(E)-3-phenylprop-2-en-(E)-ylidene]-hydrazino}-thiazol-4-yl)-phenol;
- [3-((E)-3-{[4-(4-trifluoromethylphenyl)-thiazol-2-yl]-hydrazono}-propenyl)-phenyl]-methanol;
- 3-(4-fluorophenyl)-2-propenal-[4-(4-trifluoromethylphenyl-2-thiazolyl) hydrazone;
- 3-((E)-3-{[4-(4-trifluoromethylphenyl)-thiazol-2-yl]-hydrazono}-propenyl)-benzamide;
and quite particularly 3-phenyl-2-propenal-[4-(4-trifluoromethylphenyl)-2-thiazolyl]hydrazone: 3-((E)-3-{[4-(4-trirfluoromethylphenyl)-thiazol-2-yl]-hydrazono}-propenyl)-phenol; 3-(3-methanesulphonylphenyl)-2-propenal-[4-(4-trifluoromethylphenyl-2-thiazolyl)hydrazone; and 3-(3-cyanophenyl)-2-propenal-[4-(4-trifluoromethylphenyl-2-thiazolyl)hydrazone.

12. Use of the compounds of formula I as described in any one of claims 10 or 11, or their addition salts with pharmaceutically acceptable acids, including
- 3-phenyl-2-propenal-[4-(4-nitrophenyl)-2-thiazolyl] hydrazone;
- 3-phenyl-2-propenal-[4-(4-methoxyphenyl)-2-thiazolyl] hydrazone;
- 3-phenyl-2-propenal-[4-(4-fluorophenyl)-2-thiazolyl] hydrazone;
- 3-phenyl-2-propenal-[4-(4-bromophenyl)-2-thiazolyl] hydrazone;
- 3-phenyl-2-propenal-[4-(4-chlorophenyl)-2-thiazolyl] hydrazone;
- 3-phenyl-2-propenal-[4-(4-methylphenyl)-2-thiazolyl] hydrazone;
- 3-phenyl-2-propenal-[4-(1-naphthalenyl)-2-thiazolyl] hydrazone;
- 3-phenyl-2-propenal-(5-methyl-4-phenyl-2-thiazolyl) hydrazone;
- 3-phenyl-2-propenal-[4-(2,4-dichlorophenyl)-2-thiazolyl] hydrazone;
- 3-phenyl-2-propenal-[4-(3-nitrophenyl)-2-thiazolyl] hydrazone;
- 3-phenyl-2-propenal-[4-(1,1-dimethylethyl)-2-thiazolyl] hydrazone;
- 3-phenyl-2-propenal-[4-(chlorophenyl)-5-phenyl-2-thiazolyl] hydrazone;
- 2-butenal-[4-(4-nitrophenyl)-2-thiazolyl] hydrazone;
- 3-phenyl-2-propenal-[5-(4-chlorophenyl)-4-phenyl-2-thiazolyl] hydrazone;
- 3-(4-nitrophenyl)-2-propenal-(4,5-diphenyl-2-thiazolyl) hydrazone;
- 3-phenyl-2-propenal-(4,5-diphenyl-2-thiazolyl) hydrazone;
- 2-[[3-(3-nitrophenyl)-2-propenylidene]hydrazino]-4-thiazole carboxylic acid;
- 2-[[3-(2-nitrophenyl)-2-propenylidene]hydrazino]-4-thiazole carboxylic acid;
- 3-phenyl-2-propenal-[4-chloromethyl)-2-thiazolyl] hydrazone;
- cinnamaldehyde-(4-phenyl-2-thiazolyl) hydrazone;
- crotonaldehyde-(4-phenyl-2-thiazolyl) hydrazone;
- 2-furanacroleine-(4-methyl-2-thiazolyl) hydrazone;
- cinnamaldehyde-(4-methyl-2-thiazolyl) hydrazone;
in the preparation of a medicament intended for the treatment of chronic neurodegenerative diseases.

13. Use according to claim 12, **characterized in that** the medicament is intended for the treatment of neurodegenerative polyglutamine pathologies.

14. Use according to any one of claims 12 or 13, **characterized in that** the medicament is intended for the treatment of Huntington's disease.

15. Use of the compounds of formula I, as described in any one of claims 10 or 11, or their addition salts with pharmaceutically acceptable acids, including
- 3-phenyl-2-propenal-[4-(4-nitrophenyl)-2-thiazolyl] hydrazone;
- 3-phenyl-2-propenal-[4-(4-methoxyphenyl)-2-thiazolyl] hydrazone;
- 3-phenyl-2-propenal-[4-(4-fluorophenyl)-2-thiazolyl] hydrazone;
- 3-phenyl-2-propenal-[4-(4-bromophenyl)-2-thiazolyl] hydrazone;
- 3-phenyl-2-propenal-[4-(4-chlorophenyl)-2-thiazolyl] hydrazone;
- 3-phenyl-2-propenal-[4-(4-methylphenyl)-2-thiazolyl] hydrazone;
- 3-phenyl-2-propenal-[4-(1-naphthalenyl)-2-thiazolyl] hydrazone,
- 3-phenyl-2-propenal-(5-methyl-4-phenyl-2-thiazolyl) hydrazone;
- 3-phenyl-2-propenal-[4-(2,4-dichlorophenyl)-2-thiazolyl] hydrazone;
- 3-phenyl-2-propenal-[4-(3-nitrophenyl)-2-thiazolyl] hydrazone;
- 3-phenyl-2-propenal-[4-(1,1-dimethylethyl)-2-thiazolyl] hydrazone;
- 3-phenyl-2-propenal-[4-(4-chlorophenyl)-5-phenyl-2-thiazolyl] hydrazone;
- 2-butenal-[4-(4-nitrophenyl)-2-thiazolyl] hydrazone;
- 3-phenyl-2-propenal-[5-(4-chlorophenyl)-4-phenyl-2-thiazolyl] hydrazone;
- 3-(4-nitrophenyl)-2-propenal-(4,5-diphenyl-2-thiazolyl) hydrazone;
- 3-phenyl-2-propenal-(4,5-diphenyl-2-thiazolyl) hydrazone;
- 2-[[3-(3-nitrophenyl)-2-propenylidene]hydrazino]-4-thiazole carboxylic acid
- 2-[[3-(2-nitrophenyl)-2-propenylidene]hydrazino]-4-thiazole carboxylic acid
- 3-phenyl-2-propenal-[4-chloromethyl)-2-thiazolyl] hydrazone;
- cinnamaldehyde-(4-phenyl-2-thiazolyl) hydrazone;
- crotonaldehyde-(4-phenyl-2-thiazolyl) hydrazone;
- 2-furanacroleine-(4-methyl-2-thiazolyl) hydrazone;
- cinnamaldehyde-(4-methyl-2-thiazolyl) hydrazone;
in the preparation of a medicament intended for the treatment of chronic neurodegenerative diseases, hereditary or sporadic, in particular Alzheimer's disease (AD), Parkinson's disease (PD), spinobulbar muscular atrophy or Kennedy's disease, Lewy body dementia, spinocerebellar ataxia, amyotrophic lateral sclerosis (ALS), spinal muscular atrophy (SMA), Creutzfeldt-Jakob disease, multiple sclerosis (MS), adrenoleukodystrophy, epilepsy, dementia, schizophrenia, DRPLA, neurological syndromes associated with acquired immunodeficiency syndrome (AIDS), age-related neuronal lesions, hereditary or lesional peripheral neuropathy, such as Fabry disease, Charcot-Marie-Tooth disease, Krabbe disease, leukodystrophy, diabetic neuropathies and those induced by anti-cancer treatments, traumas of the brain, peripheral nerves or spinal cord, ischemia of the brain or spinal cord following a stroke, or induced by a lack of blood irrigation, hereditary, lesional or age-related degeneration of the vision sensory neurons, such as macular degeneration and retinitis pigmentosa, or glaucoma-induced degeneration of the optic nerve, hereditary, traumatic or age-related degeneration of the auditory sensory neurons leading to a reduction in or loss of hearing.

## Patentansprüche

1. Neue chemische Verbindungen entsprechend Formel I 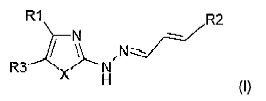 wobei
X ein Heteroatom repräsentiert, ausgewählt aus Sauerstoff, Schwefel und Stickstoff, wobei das Stickstoffatom eventuell substituiert ist durch eine Gruppe:
- ein Alkylradikal mit 1 bis 6 Kohlenstoffatomen, linear oder verzweigt, eventuell substituiert durch ein Halogenatom, ein Hydroxylradikal, eine Aminogruppe, eine Carbonsäuregruppe;
R1 eine Gruppe repräsentiert:
- ein Alkylradikal mit 1 bis 6 Kohlenstoffatomen, linear oder verzweigt, eventuell substituiert durch ein Halogenatom, ein Hydroxylradikal, eine Aminogruppe, eine Carbonsäuregruppe,
- ein Radikal, abgeleitet von einem kohlenstoffhaltigen zyklischen gesättigten Kern mit 3 bis 6 Kohlenstoffatomen, eventuell substitutiert durch ein oder mehrere Halogenatome, ein oder mehrere Alkylradikale mit 1 bis 4 Kohlenstoffatomen, linear oder verzweigt,
- ein Radikal, abgeleitet von einem kohlenstoffhaltigen aromatischen zyklischen oder polyzyklischen Kern mit 6 bis 10 Kohlenstoffatomen, eventuell ein oder mehrere Male substituiert durch ein oder mehrere Substituenten, ausgewählt aus einem Halogenatom, einem Alkylradikal mit 1 bis 4 Kohlenstoffatomen, linear oder verzweigt, eventuell substituiert durch ein oder mehrere Halogenatome, durch ein Hydroxylradikal, durch eine Aminogruppe oder durch ein Alkoxyradikal mit 1 bis 3 Kohlenstoffatomen, ein Hydroxylradikal, eine Nitrogruppe, eine Cyanogruppe, eine 1,3-Dioxolyl-Gruppe, eine Carbonylgruppe, eine Methylsulfongruppe oder eine Aminogruppe, eventuell mono- oder disubstituiert durch eine Alkylkette mit 1 bis 3 Kohlenstoffatomen, oder
- ein monozyklisches aromatisches Radikal, dessen Ring 5 oder 6 Atome aufweist und 1 oder 2 Stickstoffatome or 1 Sauerstoffatom hat;
R2 eine Gruppe repräsentiert
- ein Alkylradikal mit 1 bis 6 Kohlenstoffatomen, linear oder verzweigt, eventuell substituiert durch ein Halogenatom, ein Hydroxylradikal, eine Aminogruppe, eine Carbonsäuregruppe,
- ein Radikal, abgeleitet von einem kohlenstoffhaltigen, gesättigten, zyklischen Kern mit 3 bis 6 Kohlenstoffatomen, eventuell substituiert durch ein oder mehrere Halogenatome, ein oder mehrere Alkylradikale mit 1 bis 4 Kohlenstoffatomen, linear oder verzweigt, oder
- ein Radikal, abgeleitet von einem kohlenstoffhaltigen aromatischen zyklischen oder polyzyklischen Kern mit 6 bis 10 Kohlenstoffatomen, eventuell ein oder mehrere Male substituiert durch ein oder mehrere Substituenten, ausgewählt aus einem Halogenatom, einem Alkylradikal mit 1 bis 4 Kohlenstoffatomen, linear oder verzweigt, eventuell substituiert durch ein oder mehrere Halogenatome, durch ein Hydroxylradikal, durch eine Aminogruppe oder durch ein Alkoxyradikal mit 1 bis 3 Kohlenstoffatomen, ein Hydroxylradikal, eine Nitrogruppe, eine Cyanogruppe, eine 1,3-Dioxolyl-Gruppe, eine Carbonylgruppe, eine Methylsulfongruppe oder eine Aminogruppe, eventuell mono- oder disubstituiert durch eine Alkylkette mit 1 bis 3 Kohlenstoffatomen, oder
- ein monozyklisches aromatisches Radikal, dessen Ring 5 oder 6 Atome aufweist und 1 oder 2 Stickstoffatome umfasst;
R3 ein Wasserstoffatom oder eine Gruppe repräsentiert:
- ein Alkylradikal mit 1 bis 6 Kohlenstoffatomen, linear oder verzweigt, eventuell substituiert durch ein Halogenatom, ein Hydroxylradikal, eine Aminogruppe, eine Carbonsäuregruppe, oder
- ein Radikal, abgeleitet von einem kohlenstoffhaltigen aromatischen zyklischen oder polyzyklischen Kern mit 6 bis 10 Kohlenstoffatomen, eventuell ein oder mehrere Male substituiert durch ein oder mehrere Substituenten, ausgewählt aus einem Halogenatom, einem Alkylradikal mit 1 bis 4 Kohlenstoffatomen, linear oder verzweigt, eventuell substituiert durch ein oder mehrere Halogenatome, durch ein Hydroxyradikal, durch eine Aminogruppe oder durch ein Alkoxyradikal mit 1 bis 3 Kohlenstoffatomen, ein Hydroxylradikal, eine Nitrogruppe, eine Cyanogruppe, eine 1,3-Dioxolyl-Gruppe, eine Carbonylgruppe, eine Methylsulfongruppe oder eine Aminogruppe, eventuell mono- oder disubstituiert durch eine Alkylkette mit 1 bis 3 Kohlenstoffatomen;
mit Ausnahme der folgenden Verbindungen:
- 3-Phenyl-2-propenal-[4-(4-nitrophenyl)-2-thiazolyl] hydrazon;
- 3-Phenyl-2-propenal-[4-(4-methoxyphenyl)-2-thiazolyl] hydrazon;
- 3-Phenyl-2-propenal-[4-(4-fluorphenyl)-2-thiazolyl] hydrazon;
- 3-Phenyl-2-propenal-[4-(4-bromphenyl)-2-thiazolyl] hydrazon;
- 3-Phenyl-2-propenal-[4-(4-chlorphenyl)-2-thiazolyl] hydrazon;
- 3-Phenyl-2-propenal-[4-(4-methylphenyl)-2-thiazolyl] hydrazon;
- 3-Phenyl-2-propenal-[4-(1-naphthalenyl)-2-thiazolyl] hydrazon;
- 3-Phenyl-2-propenal-(5-methyl-4-phenyl-2-thiazolyl) hydrazon;
- 3-Phenyl-2-propenal-[4-(2,4-dichlorphenyl)-2-thiazolyl] hydrazon;
- 3-Phenyl-2-propenal-[4-(3-nitrophenyl)-2-thiazolyl] hydrazon;
- 3-Phenyl-2-propenal-[4-(1,1-dimethylethyl)-2-thiazolyl] hydrazon;
- 3-Phenyl-2-propenal-[4-(4-chlorphenyl)-5-phenyl-2-thiazolyl] hydrazon;
- 2-Butenal-[4-(4-nitrophenyl)-2-thiazolyl] hydrazon;
- 3-Phenyl-2-propenal-[5-(4-chlorphenyl)-4-phenyl-2-thiazolyl] hydrazon;
- 3-(4-Nitrophenyl)-2-propenal-(4,5-diphenyl-2-thiazolyl) hydrazon;
- 3-Phenyl-2-propenal-(4,5-diphenyl-2-thiazolyl) hydrazon;
- 2-[[3-(3-Nitrophenyl)-2-propenyliden]hydrazino]-4-thiazol-carbonsäure;
- 2-[[3-(2-Nitrophenyl)-2-propenyliden]hydrazino]-4-thiazol-carbonsäure;
- 3-Phenyl-2-propenal-[4-chlormethyl)-2-thiazolyl] hydrazon;
- Zimtaldehyd-(4-phenyl-2-thiazolyl) hydrazon;
- Krotonaldehyd-(4-phenyl-2-thiazolyl) hydrazon;
- 2-Furanacrolein-(4-methyl-2-thiazolyl) hydrazon;
- Zimtaldehyd-(4-methyl-2-thiazolyl) hydrazon;
sowie deren Additionssalze mit den pharmazeutisch akzeptablen Säuren.

2. Verbindung der Formel I nach Anspruch 1, **dadurch gekennzeichnet, dass** X ein Schwefelatom ist.

3. Verbindung der Formel I nach Anspruch 1 oder 2, **dadurch gekennzeichnet, dass** R1 ein Phenylradikal ist, substituiert in der para-Position durch ein Chloratom, durch eine Methyl- oder Trifluormethylgruppe, oder in der meta-Position durch eine Hydroxylgruppe, oder ein Phenylradikal, disubstituiert in der ortho-Position durch eine Methoxygruppe und in der para-Position durch ein Chloratom oder in der ortho-Position durch eine Methylgruppe und in der para-Position durch eine Hydroxylgruppe oder in der meta-Position durch ein Fluoratom oder in der para-Position durch ein Chloratom.

4. Verbindung der Formel I nach irgendeinem der Ansprüche 1 bis 3, **dadurch gekennzeichnet, dass** R2 ein Pyridin-4-yl, ein Pyridin-2-yl oder Pyridin-3-yl Radikal oder ein Benzo[1,3]dioxol-5-yl Radikal oder ein Phenylradikal ist, oder Phenyl, substituiert durch eine Nitro-, Cyano-, Methoxy-, Amino-, Methylsulfon-, Amin-, Methylketon-, Hydroxymethyl- oder Hydroxylgruppe in der meta-Position, oder Phenyl, substituiert in der ortho-Position durch eine Methoxygruppe, Methyl, ein Fluor- oder Chloratom, oder Phenyl, substituiert in der para-Position durch ein Fluoratom.

5. Verbindung der Formel I nach irgendeinem der Ansprüche 1 bis 4, **dadurch gekennzeichnet, dass** R3 ein Wasserstoffatom oder eine Methylgruppe ist

6. Verbindung der Formel I nach irgendeinem der Ansprüche 1 bis 5, **dadurch gekennzeichnet, dass**
- X ein Schwefelatom repräsentiert,
- R3 ein Wasserstoffatom ist,
- R1 ein Phenylradikal ist, substituiert in der para-Position durch ein Chloratom, durch eine Trifluormathylgruppe, oder in der meta-Position durch eine Hydroxylgruppe, oder ein Phenylradikal, disubstituiert in der ortho-Position durch eine Methoxygruppe und in der para-Position durch ein Chloratom, oder in der ortho-Position durch eine Methylgruppe und in der para-Position durch eine Hydroxylgruppe, oder in der meta-Position durch ein Fluoratom und in der para-Position durch ein Chloratom,
- R2 ein Pyridin-4-yl, Pyridin-2-yl oder Pyridin-3-yl Radikal oder ein Benzo[1,3]dioxol-5-yl Radikal oder ein Phenylradikal, oder Phenyl, substituiert durch eine Nitro-, Cyano-, Amino-, Methylsulfon-, Amid-, Methylketon-, Hydroxymethylhydroxyl- oder Methoxygruppe in der meta-Position, oder Phenyl, substituiert in der ortho-Position durch eine Methoxygruppe, Methyl, ein Fluor-oder Chloratom, oder Phenyl, substituiert in der para-Position durch ein Fluoratom, ist.

7. Verbindung der Formel I nach irgendeinem der Ansprüche 1 bis 6, **dadurch gekennzeichnet, dass** es sich um Folgendes handelt:
- 3-(2-Fluorphenyl)-2-propenal-[4-(4-chlorphenyl-2-thiazolyl) hydrazon;
- 3-(3-Nitrophenyl)-2-propenal-[4-(4-chlorphenyl-2-thiazolyl) hydrazon;
- 3-(2-Methoxyphenyl)-2-propenal-[4-(4-chlorphenyl-2-thiazolyl) hydrazon,
- 3-(3-Methoxyphenyl)-2-propenal-[4-(4-chlorphenyl-2-thiazolyl) hydrazon;
- 3-Phenyl-2-propenal-[4-(3-hydroxyphenyl-2-thiazolyl) hydrazon;
- 3-Pyridin-4-yl-2-propenal-[4-(4-chlorphenyl-2-thiazolyl) hydrazon;
- 3-(2-Methylphenyl)-2-propenal-[4-(4-chlorphenyl-2-thiazolyl) hydrazon;
- 3-(3-Cyanophenyl)-2-propenal-[4-(4-chlorphenyl-2-thiazolyl) hydrazon;
- 3-(2-Chlorphenyl)-2-propenal-[4-(4-chlorphenyl-2-thiazolyl) hydrazon;
- 3-(3-Aminophenyl)-2-propenal-[4-(4chlorphenyl-2-thiazolyl) hydrazon;
- 3-(3-Nitrophenyl)-2-propenal-[4-(4-trifluormethylphenyl-2-thiazolyl) hydrazon;
- 3-((E)-3-{[4-(4-Chlorphenyl)-thiazol-2-yl]-hydrazono}-propenyl)-phenol;
- 3-Pyridin-4-yl-2-propenal-[4-(4-trifluormethylphenyl-2-thiazolyl) hydrazon;
- 3-(3-Aminophenyl)-2-propenal-[4-(4-trifluormethylphenyl-2-thiazolyl) hydrazon;
- 3-(3-Methansulfonylphenyl)-2-propenal-[4-(4-chlorphenyl-2-thiazolyl) hydrazon;
- 3-(3-Cyanophenyl)-2-propenal-[4-(4-hydroxyphenyl-2-thiazolyl) hydrazon;
- 3-(3-Benzo[1,3]dioxol-5-yl)-2-propenal-[4-(4-trifluormethylphenyl-2-thiazolyl) hydrazon;
- 3-Phenyl-2-propenal-[4-(4-chloro-2-methoxyphenyl-2-thiazolyl) hydrazon;
- 1-[3-((E)-3-{[4-(4-Trifluormethylphenyl)-thiazol-2-yl]-hydrazono]-propenyl)-phenyl]-ethanon;
- 3-Pyridin-2-yl-2-propenal-[4-(4-chlorphenyl-2-thiazolyl) hydrazon;
- 3-Pyridin-2-yl-2-propenal-[4-(4-trifluormethylphenyl-2-thiazolyl) hydrazon;
- 3-Methyl-4-(2-{N'-[(E)-3-phenylprop-2-en-(E)-yliden]-hydrazino}-thiazol-4-yl)-phenol;
- [3-((E)-3-{[4-(4-Trifluormethylphenyl)-thiazol-2-yl]-hydrazono}-propenyl)-phenyl]-methanol;
- 3-(4-Fluorphenyl)-2-propenal-[4-(4-trifluormethylphenyl-2-thiazolyl) hydrazon;
- 3-((E)-3-{[4-(4-Trifluormethylphenyl)-thiazol-2-yl]-hydrazonorpropenyl)-benzamid;
und ganz besonders 3-Phenyl-2-propenal-[4-(4-trifluormethylphenyl)-2-thiazolyl] hydrazon; 3-((E)-3-{[4-(4-Trifluormethylphenyl)-thiazol-2-yl]-hydrazono}-propenyl)-phenol; 3-(3-Methansulfonylphenyl)-2-propenal-[4-(4-trifluormethylphenyl-2-thiazolyl)hydrazon, und 3-(3-Cyanophenyl)-2-propenal-[4-(4-trifluoromethylphenyl-2-thiazolyl)hydrazon.

8. Verfahren zur Herstellung einer Verbindung der Formel I gemäß Beschreibung in Anspruch 1, bei der X ein Schwefelatom repräsentiert, R1, R2 und R3 die oben in den Ansprüchen 1 bis 7 beschriebenen Bedeutungen haben, bei dem ein α-Bromketon der Formel II 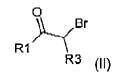 wobei R1 und R3 die oben angegebenen Bedeutungen haben können, mit einer Verbindung der Formel III 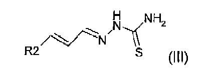 reagiert wird, wobei R2 die oben angegebene Bedeutung haben kann, in einem Minimum an Lösungsmittel, so gewählt, dass die gewünschte Verbindung der Formel I erhalten wird.

9. Zusammensetzung, insbesondere eine pharmazeutische oder medikamentöse Zusammensetzung, die wenigstens eine Verbindung der Formel I wie in einem der Ansprüche 1 bis 7 beschrieben umfasst, mit Ausnahme der folgenden Verbindungen:
- 3-Phenyl-2-propenal-[4-(4-nitrophenyl)-2-thiazolyl] hydrazon;
- 3-Phenyl-2-propenal-[4-(4-methoxyphenyl)-2-thiazolyl] hydrazon;
- 3-Phenyl-2-propenal-[4-(4-fluorphenyl)-2-thiazolyl] hydrazon;
- 3-Phenyl-2-propenal-[4-(4-bromphenyl)-2-thiazolyl] hydrazon;
- 3-Phenyl-2-propenal-[4-(4-chlorphenyl)-2-thiazolyl] hydrazon;
- 3-Phenyl-2-propenal-[4-(4-methylphenyl)-2-thiazolyl] hydrazon;
- 3-Phenyl-2-propenal- [4-(1-naphthalenyl)-2-thiazolyl] hydrazon;
- 3-Phenyl-2-propenal-(5-methyl-4-phenyl-2-thiazolyl) hydrazon;
- 3-Phenyl-2-propenal-[4-(2,4-dichlorphenyl)-2-thiazolyl] hydrazon;
- 3-Phenyl-2-propenal-[4-(3-nitrophenyl)-2-thiazolyl] hydrazon,
- 3-Phenyl-2-propenal-[4-(1,1-dimethylethyl)-2-thiazolyl] hydrazon;
- 3-Phenyl-2-propenal-[4-(4-chlorphenyl)-5-phenyl-2-thiazolyl] hydrazon;
- 2-Butenal-[4-(4-nitrophenyl)-2-thiazolyl] hydrazon,
- 3-Phenyl-2-propenal-[5-(4-chlorphenl)-4-phenyl-2-thiazolyl] hydrazon;
- 3-(4-Nitrophenyl)-2-propenal-(4,5-diphenyl-2-thiazolyl) hydrazon;
- 3-Phenyl-2-propenal-(4,5-diphenyl-2-thiazolyl) hydrazon;
- 2-[[3-(3-Nitrophenyl)-2-propenyliden]hydrazino]-4-thiazol-carbonsäure;
- 2-[[3-(2-Nitrophenyl)-2-propenyliden]hydrazino]-4-thiazol-carbonsäure;
- 3-Phenyl-2-propenal-[4-chlormethyl)-2-thiazolyl] hydrazon;
- Zimtaldehyd-(4-phenyl-2-thiazolyl) hydrazon;
- Krotonaldehyd-(4-phenyl-2-thiazolyl) hydrazon;
- 2-Furanacrolein-(4-methyl-2-thiazolyl) hydrazon;
- Zimtaldehyd-(4-methyl-2-thiazolyl) hydrazon;
sowie deren Additionssalze mit den pharmazeutisch akzeptablen Säuren.

10. Verwendung von Verbindungen der Formel I 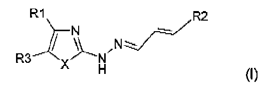 wobei X, R1, R2 und R3 die oben in den Ansprüchen 1 bis 7 angegebenen Bedeutungen haben können, einschließlich
- 3-Phenyl-2-propenal-[4-(4-nitrophenyl)-2-thiazolyl] hydrazon;
- 3-Phenyl-2-propenal-[4-(4-methouyphenyl)-2-thiazolyl] hydrazon;
- 3-Phenyl-2-propenal-[4-(4-fluorphenyl)-2-thiazolyl] hydrazon;
- 3-Phenyl-2-propenal-[4-(4-bromphenyl)-2-thiazolyl] hydrazon;
- 3-Phenyl-2-propenal-[4-(4-chlorphenyl)-2-thiazolyl] hydrazon;
- 3-Phenyl-2-propenal-[4-(4-methylphenyl)-2-thiazolyl] hydrazon;
- 3-Phenyl-2-propenal- [4-(1-naphthalenyl)-2-thiazolyl] hydrazon;
- 3-Phenyl-2-propenal-(5-Methyl-4-phenyl-2-thiazolyl) hydrazon;
- 3-Phenyl-2-propenal-[4-(2,4-dichlorphenyl)-2-thiazolyl] hydrazon;
- 3-Phenyl-2-propenal-[4-(3-nitrophenyl)-2-thiazolyl] hydrazon;
- 3-Phenyl-2-propenal-[4-(1,1-dimethylethyl)-2-thiazolyl] hydrazon;
- 3-Phenyl-2-propenal-[4-(4-chlorphenyl)-5-phenyl-2-thiazolyl] hydrazon;
- 2-Butenal-[4-(4-nitrophenyl)-2-thiazolyl] hydrazon;
- 3-Phenyl-2-propenal-[5-(4-chlorphenyl)-4-phenyl-2-thiazolyl] hydrazon;
- 3-(4-Nitrophenyl)-2-propenal-(4,5-diphenyl-2-thiazolyl) hydrazon;
- 3-Phenyl-2-propenal-(4,5-diphenyl-2-thiazolyl) hydrazon;
- 2-[[3-(3-Nitrophenyl)-2-propenyliden]hydrazino]-4-thiazol-carbonsäure;
- 2-[[3-(2-Nitrophenyl)-2-propenyliden]hydrazino]-4-thiazol-carbonsäure;
- 3-Phenyl-2-propenal-[4-chlormethyl)-2-thiazolyl] hydrazon;
- Zimtaldehyd-(4-phenyl-2-thiazolyl) hydrazon;
- Krotonaldehyd-(4-phenyl-2-thiazolyl) hydrazon;
- 2-Furanacrolein-(4-methyl-2-thiazolyl) hydrazon;
bei der Herstellung eines Medikaments.

11. Verwendung nach Anspruch 10, **dadurch gekennzeichnet, dass** die Verbindung der Formel I ausgewählt ist aus:
- 3-(2-Fluorphenyl)-2-propenal-[4-(4-chlorphenyl-2-thiazolyl) hydrazon;
- 3-(3-Nitrophenyl)-2-propenal-[4-(4-chlorphenyl-2-thiazolyl) hydrazon;
- 3-(2-Methoxyphenyl)-2-propenal-[4-(4-chlorphenyl-2-thiazolyl) hydrazon;
- 3-(3-Methoxyphenyl)-2-propenal-[4-(4-chlorphenyl-2-thiazolyl) hydrazon;
- 3-Phenyl-2-propenal-[4-(3-hydroxyphenyl-2-thiazolyl) hydrazon;
- 3-Pyridin-4-yl-2-propenal-[4-(4-chlorphenyl-2-thiazolyl) hydrazon;
- 3-(2-Methylphenyl)-2-propenal-[4-(4-chlorphenyl-2-thiazolyl) hydrazon;
- 3-(3Cyanophenyl)-2-propenal-[4-(4-chlorphenyl-2-thiazolyl) hydrazon;
- 3-(2-Chlorphenyl)-2-propenal-[4-(4-chlorphenyl-2-thiazolyl) hydrazon;
- 3-(3-Aminophenyl)-2-propenal-[4-(4-chlorphenyl-2-thiazolyl) hydrazon;
- 3-(3-Nitrophenyl)-2-propenal-[4-(4-trifluormethylphenyl-2-thiazolyl) hydrazon;
- 3-((E)-3-{[4-(4-Chlorphenyl)-thiazol-2-yl]-hydrazono}-propenyl)-phenol;
- 3-Pyridin-4-yl-2-propenal-[4-(4-trifluormethylphenyl-2-thiazolyl) hydrazon;
- 3-(3-Aminophenyl)-2-propenal-[4-(4-trifluormethylphenyl-2-thiazolyl) hydrazon;
- 3-(3-Methansulfonylphenyl)-2-propenal-[4-(4-chlorphenyl-2-thiazolyl) hydrazon;
- 3-(3-Cyanophenyl)-2-propenal-[4-(4-hydroxyphenyl-2-thiazolyl) hydrazon;
- 3-(3-Benzo[1,3]dioxol-5-yl)-2-propenal-[4-(4-trifluormethylphenyl-2-thiazolyl) hydrazon;
- 3-Phenyl-2-propenal-[4-(4-chloro-2-methoxyphenyl-2-thiazolyl) hydrazon;
- 1-[3-((E)-3-{[4-(4-Trifluormethylphenyl)-thiazol-2-yl]-hydrazono}-propenyl)-phenyl]-ethanon;
- 3-Pyridin-2-yl-2-propenal-[4-(4-chlorphenyl-2-thiazolyl) hydrazon;
- 3-Pyridin-2-yl-2-propenal-[4-(4-trifluormethylphenyl-2-thiazolyl) hydrazon;
- 3-Methyl-4-(2-{N'-[(E)-3-phenylprop-2-en-(E)-yliden]-hydrazino}-thiazol-4-yl)-phenol;
- [3((E)-3-{[4-(4-Trifluormethylphenyl)-thiazol-2-yl]-hydrazono}-propenyl)-phenyl]-methanol;
- 3-(4-Fluorphenyl)-2-propenal-[4-(4-trifluormethylphenyl-2-thiazolyl) hydrazon;
- 3-((E)-3-{[4-(4-Trifluormethylphenyl)-thiazol-2-yl]-hydrazono}-propenyl)-benzamid;
und ganz besonders 3-Phenyl-2-propenal-[4-(4-trifluormethylphenyl)-2-thiazolyl]hydrazon; 3-((E)-3-{[4-(4-Trifluormethylphenyl)-thiazol-2-yl]-hydrazono}-propenyl)-phenol; 3-(3-Methansulfonylphenyl)-2-propenal-[4-(4-trifluormethylphenyl-2-thiazolyl)hydrazon; und 3-(3-Cyanophenyl)-2-propenal-[4-(4-trifluormethylphenyl-2-thiazolyl)hydrazon

12. Verwendung von Verbindungen der Formel I gemäß Beschreibung in Anspruch 10 oder 11 oder deren Additionssalze mit den pharmazeutisch akzeptablen Säuren, einschließlich
- 3-Phenyl-2-propenal-[4-(4-nitrophenyl)-2-thiazolyl] hydrazon;
- 3-Phenyl-2-propenal-[4-(4-methoxyphenyl)-2-thiazolyl] hydrazon;
- 3-Phenyl-2-propenal-[4-(4-fluorphenyl)-2-thiazolyl] hydrazon,
- 3-Phenyl-2-propenal-[4-(4-bromphenyl)-2-thiazolyl] hydrazon;
- 3-Phenyl-2-propenal-[4-(4-chlorphenyl)-2-thiazolyl] hydrazon;
- 3-Phenyl-2-propenal-[4-(4-methylphenyl)-2-thiazolyl] hydrazon;
- 3-Phenyl-2-propenal-[4-(1-naphthalenyl)-2-thiazolyl] hydrazon;
- 3-Phenyl-2-propenal-(5-methyl-4-phenyl-2-thiazolyl) hydrazon;
- 3-Phenyl-2-propenal-[4-(2,4-dichlorphenyl)-2-thiazolyl] hydrazon;
- 3-Phenyl-2-propenal-[4-(3-nitrophenyl)-2-thiazolyl] hydrazon;
- 3-Phenyl-2-propenal-[4-(1,1-dimethylethyl)-2-thiazolyl] hydrazon;
- 3-Phenyl-2-propenal-[4-(4-chlorphenyl)-5-phenyl-2-thiazolyl] hydrazon;
- 2-Butenal-[4-(4-nitrophenyl)-2-thiazolyl] hydrazon;
- 3-Phenyl-2-propenal-[5-(4-chlorphenyl)-4-phenyl-2-thiazolyl] hydrazon;
- 3-(4-Nitrophenyl)-2-propenal-(4,5-diphenyl-2-thiazolyl) hydrazon;
- 3-Phenyl-2-propenal-(4,5-diphenyl-2-thiazolyl) hydrazon;
- 2-[[3-(3-Nitrophenyl)-2-propenyliden]hydrazino]-4-thiazol-carbonsäure;
- 2-[[3-(2-Nitrophenyl)-2-propenyliden]hydrazino]-4-thiazol-carbonsäure;
- 3-Phenyl-2-propenal-[4-chlormethyl)-2-thiazolyl] hydrazon;
- Zimtaldehyd-(4-phenyl-2-thiazolyl) hydrazon;
- Krotonaldehyd-(4-phenyl-2-thiazolyl) hydrazon;
- 2-Furanacrolein-(4-methyl-2-thiazolyl) hydrazon;
- Zimtaldehyd-(4-methyl-2-thiazolyl) hydrazon;
bei der Herstellung eines Medikaments zur Behandlung von chronischen neurodegenerativen Krankheiten.

13. Verwendung nach Anspruch 12, **dadurch gekennzeichnet, dass** das Medikament für die Behandlung von neurodegenerativen Polyglutaminkrankheiten bestimmt ist.

14. Verwendung nach Anspruch 12 oder 13, **dadurch gekennzeichnet, dass** das Medikament für die Behandlung der Huntington'schen Krankheit bestimmt ist.

15. Verwendung von Verbindungen der Formel I gemäß Beschreibung in Anspruch 10 oder 11 oder deren Additionssalze mit den pharmazeutisch akzeptablen Säuren, einschließlich:
- 3-Phenyl-2-propenal-[4-(4-nitrophenyl)-2-thiazolyl] hydrazon;
- 3-Phenyl-2-propenal-[4-(4-methoxyphenyl)-2-thiazolyl] hydrazon;
- 3-Phenyl-2-propenal-[4-(4-fluorphenyl)-2-thiazolyl] hydrazon;
- 3-Phenyl-2-propenal-[4-(4-bromphenyl)-2-thiazolyl] hydrazon;
- 3-Phenyl-2-propenal-[4-(4-chlorphenyl)-2-thiazolyl] hydrazon;
- 3-Phenyl-2-propenal-[4-(4-methylphenyl)-2-thiazolyl] hydrazon;
- 3-Phenyl-2-propenal-[4-(1-naphthalenyl)-2-thiazolyl] hydrazon;
- 3-Phenyl-2-propenal-(5-methyl-4-phenyl-2-thiazolyl) hydrazon;
- 3-Phenyl-2-propenal-[4-(2,4-dichlorphenyl)-2-thiazolyl] hydrazon;
- 3-Phenyl-2-propenal-[4-(3-nitrophenyl)-2-thiazolyl] hydrazon;
- 3-Phenyl-2-propenal-[4-(1,1-dimethylethyl)-2-thiazolyl] hydrazon;
- 3-Phenyl-2-propenal-[4-(4-chlorphenyl)-5-phenyl-2-thiazolyl] hydrazon;
- 2-Butenal-[4-(4-nitrophenyl)-2-thiazolyl] hydrazon;
- 3-Phenyl-2-propenal-[5-(4-chlorphenyl)-4-phenyl-2-thiazolyl] hydrazon;
- 3-(4-Nitrophenyl)-2-propenal-(4,5-diphenyl-2-thiazolyl) hydrazon;
- 3-Phenyl-2-propenal-(4,5-diphenyl-2-thiazolyl) hydrazon;
- 2-[[3-(3-Nitrophenyl)-2-propenyliden]hydrazino]-4-thiazol-carbonsäure 2-[[3-(2-Nitrophenyl)-2-propenyliden]hydrazino]-4-thiazol-carbonsäure
- 3-Phenyl-2-propenal[4-chlormethyl)-2-thiazolyl] hydrazon;
- Zimtaldehyd-(4-phenyl-2-thiazolyl) hydrazon:
- Krotonaldehyd-(4-phenyl-2-thiazolyl) hydrazon;
- 2-Furanacrolein-(4-methyl-2-thiazolyl) hydrazon;
- Zimtaldehyd-(4-methyl-2-thiazolyl) hydrazon;
bei der Herstellung eines Medikaments zur Behandlung von chronischen neurodegenerativen Krankheiten, erblich oder sporadisch, insbesondere die Alzheimer-Krankheit (AD), die Parkinson'sche Krankheit (PD), spinobulbäre Muskelatrophie oder die KENNEDY-Krankheit, Lewy-Körper-Demenz, spinozerebelläre Ataxien, amyotrophe Lateralsklerose (ALS), spinale Amyotrophien (SMA), die Greutzfeldt-Jakob-Krankheit, multiple Sklerose (MS), Adrenoleukodystrophie, Epilepsie, Demenzen, Schizophrenie, DRPLA, neurologische Syndrome in Verbindung mit dem erworbenen Immundetektsyndrom (AIDS), alterungsbedingte neuronale Läsionen, ererbte oder läsionelle periphere Neuropathien wie die Krankheiten gemäß Fabry, Charcot-Marie-Tooth, Krabbe, Leukodystrophien, diabetische Neuropathien und solche, die durch Anti-Krebsbehandlungen induziert werden, Traumata des Gehirns, der peripheren Nerven oder des Rückenmarks, Ischämien des Gehirns oder des Rückenmarks nach einem zerebrovaskulären Unfall oder aufgrund von Durchblutungsmangel, ererbte, läsionelle oder mit dem Altern verbundene Degenerationen der sensorischen Neuronen des Sehens, wie Makuladegenerationen, Retinitis pigmentos oder Degenerationen des Sehnervs, verursacht durch Glaukome, ererbte, traumatische oder mit dem Altern verbundene Degenerationen der sensorischen Neuronen des Hörens, die eine Verringerung oder den Verlust des Hörens mit sich bringen.
